# EUROPEAN PATENT APPLICATION

(11) **EP 2 050 763 A2**
(43) Date of publication of application: **22.04.2009**
(21) Application number: 08021786.2
(22) Date of filing: 09.03.2006
(51) Int. Cl.: C07K 14/47, C12N 15/11, A61K 31/713

(54) **Methods and compositions for modulating vascular integrity**

(30) Priority: 10.03.2005 US 660172 P
(62) Divisional of application: 06737484.3
(71) Applicant: GENENTECH, INC., South San Francisco CA 94080 (US)
(72) Inventor: Gerber, Hans-Peter, SE Bellevue WA 98006 (US); Hesser, Boris A., San Fancisco CA 94133 (US)
(74) Representative: Tollervey, Rebecca Marie

(57) **Abstract**

The invention provides methods and compositions useful for modulating vascular integrity

Sequence Listing

<110> Genentech, Inc.

<120> METHODS AND COMPOSITIONS FOR MODULATING VASCULAR INTEGRITY

<130> RXT/FP6572879

<140> Divisional of EP 06737484.3
<141> 2006-03-09

<150> US 60/660,172
<151> 2005-03-10

<160> 33

<210> 1
<211> 197
<212> PRT
<213> Homo sapiens

<400> 1
Met His Phe Arg Asn Phe Asn Tyr Ser Phe Ser Ser Leu Ile Ala
1               5                   10                  15

Cys Val Ala Asn Ser Asp Ile Phe Ser Glu Ser Glu Thr Arg Ala
                20                  25                  30

Lys Phe Glu Ser Leu Phe Arg Thr Tyr Asp Lys Asp Ile Thr Phe
                35                  40                  45

Gln Tyr Phe Lys Ser Phe Lys Arg Val Arg Ile Asn Phe Ser Asn
                50                  55                  60

Pro Phe Ser Ala Ala Asp Ala Arg Leu Gln Leu His Lys Thr Glu
                65                  70                  75

Phe Leu Gly Lys Glu Met Lys Leu Tyr Phe Ala Gln Thr Leu His
                80                  85                  90

Ile Gly Ser Ser His Leu Ala Pro Pro Asn Pro Asp Lys Gln Phe
                95                  100                 105

Leu Ile Ser Pro Pro Ala Ser Pro Pro Val Gly Trp Lys Gln Val
                110                 115                 120

Glu Asp Ala Thr Pro Val Ile Asn Tyr Asp Leu Leu Tyr Ala Ile
                125                 130                 135

Ser Lys Leu Gly Pro Gly Glu Lys Tyr Glu Leu His Ala Ala Thr
                140                 145                 150

Asp Thr Thr Pro Ser Val Val Val His Val Cys Glu Ser Asp Gln
                155                 160                 165

Glu Lys Glu Glu Glu Glu Glu Met Glu Arg Met Arg Arg Pro Lys
                170                 175                 180

Pro Lys Ile Ile Gln Thr Arg Arg Pro Glu Tyr Thr Pro Ile His
                185                 190                 195

Leu Ser


<210> 2
<211> 19
<212> RNA
<213> Artificial sequence

<220>
<223> sequence is synthesized

<400> 2
gcucagaccu  uacacauag 19

<210> 3
<211> 19
<212> RNA
<213> Artificial sequence

<220>
<223> sequence is synthesized

<400> 3
ggacaucacc  uuucaguau 19

<210> 4
<211> 19
<212> RNA
<213> Artificial sequence

<220>
<223> sequence is synthesized

<400> 4
gaaagaauga  ggagaccua 19

<210> 5
<211> 19
<212> RNA
<213> Artificial sequence

<220>
<223> sequence is synthesized

<400> 5
gacaucaccu  uucaguauu 19

<210> 6
<211> 21

<212> RNA
<213> Artificial sequence

<220>
<223> sequence is synthesized

<400> 6
aacguaugac aaggacauca c 21

<210> 7
<211> 21
<212> RNA
<213> Artificial sequence

<220>
<223> sequence is synthesized

<400> 7
aaggacauca ccuuucagua u 21

<210> 8
<211> 21
<212> RNA
<213> Artificial sequence

<220>
<223> sequence is synthesized

<400> 8
aaguuauauu uugcucagac c 21

<210> 9
<211> 21
<212> RNA
<213> Artificial sequence

<220>
<223> sequence is synthesized

<400> 9
aagaugcgac cccagucaua a 21

<210> 10
<211> 19
<212> RNA
<213> Artificial sequence

<220>
<223> sequence is synthesized

<400> 10
cguaugagcu ucggauuga 19

<210> 11
<211> 18

<212> RNA
<213> Artificial sequence

<220>
<223> sequence is synthesized

<400> 11
gcagagcacg gacagcua 18

<210> 12
<211> 19
<212> RNA
<213> Artificial sequence

<220>
<223> sequence is synthesized

<400> 12
ucagaaacuc cgacauuga 19

<210> 13
<211> 19
<212> RNA
<213> Artificial sequence

<220>
<223> sequence is synthesized

<400> 13
gccggaaucc ugaaacuca 19

<210> 14
<211> 29
<212> DNA
<213> Artificial sequence

<220>
<223> sequence is synthesized

<400> 14
aggttgtgaa aacagcagca atgcaatgt 29

<210> 15
<211> 19
<212> DNA
<213> Artificial sequence

<220>
<223> sequence is synthesized

<400> 15
ccacaggaag ccgcctagt 19

<210> 16
<211> 21

<212> DNA
<213> Artificial sequence

<220>
<223> sequence is synthesized

<400> 16
tgagggaaga aaggaaacgc t 21

<210> 17
<211> 22
<212> DNA
<213> Artificial sequence

<220>
<223> sequence is synthesized

<400> 17
aggtccaggc cactcccacc gt 22

<210> 18
<211> 19
<212> DNA
<213> Artificial sequence

<220>
<223> sequence is synthesized

<400> 18
gtcatcttgg agggaccaa 19

<210> 19
<211> 19
<212> DNA
<213> Artificial sequence

<220>
<223> sequence is synthesized

<400> 19
ttctgccatg cctgtatca 19

<210> 20
<211> 40
<212> DNA
<213> Artificial sequence

<220>
<223> sequence is synthesized

<400> 20
ttaactgact taagtggcat taaacatttg agagctaagt 40

<210> 21
<211> 26

<212> DNA
<213> Artificial sequence

<220>
<223> sequence is synthesized

<400> 21
cagcttccta atatgcttta caatct 26

<210> 22
<211> 27
<212> DNA
<213> Artificial sequence

<220>
<223> sequence is synthesized

<400> 22
caatgtcaac catagaagct ttaagta 27

<210> 23
<211> 25
<212> DNA
<213> Artificial sequence

<220>
<223> sequence is synthesized

<400> 23
cctggctacc tgcatgctgt tccgt 25

<210> 24
<211> 21
<212> DNA
<213> Artificial sequence

<220>
<223> sequence is synthesized

<400> 24
cagtaggtgc attggaatca a 21

<210> 25
<211> 24
<212> DNA
<213> Artificial sequence

<220>
<223> sequence is synthesized

<400> 25
tcttagcaaa atggctaaaa gaag 24

<210> 26
<211> 24

<212> DNA
<213> Artificial sequence

<220>
<223> sequence is synthesized

<400> 26
atccctgctc aacccctctg gagt 24

<210> 27
<211> 18
<212> DNA
<213> Artificial sequence

<220>
<223> sequence is synthesized

<400> 27
cccaggtcct tgtttgct 18

<210> 28
<211> 19
<212> DNA
<213> Artificial sequence

<220>
<223> sequence is synthesized

<400> 28
tccaaccgtc attgaaacc 19

<210> 29
<211> 29
<212> DNA
<213> Artificial sequence

<220>
<223> sequence is synthesized

<400> 29
cgaagtttgt tcatcagact cctgtgcgt 29

<210> 30
<211> 20
<212> DNA
<213> Artificial sequence

<220>
<223> sequence is synthesized

<400> 30
gcatggtacg gagatgtttc 20

<210> 31
<211> 18

<212> DNA
<213> Artificial sequence

<220>
<223> sequence is synthesized

<400> 31
ggccacattg ggaaagtt 18

<210> 32
<211> 36
<212> DNA
<213> Artificial sequence

<220>
<223> sequence is synthesized

<400> 32
ccgaattccc atgcatttta gaaactttaa ctacag 36

<210> 33
<211> 37
<212> DNA
<213> Artificial sequence

<220>
<223> sequence is synthesized

<400> 33
gggctcgagc tagctgaggt ggatcggcgt gtactcc 37

## Description

### RELATED APPLICATIONS

This application is a non-provisional application filed under 37 CFR 1.53(b)(1), claiming priority under 35 USC 119(e) to provisional application number 60/660,172 filed March 10, 2005, the contents of which are incorporated herein by reference.

### TECHNICAL FIELD

The present invention relates generally to the fields of treatment of diseases and other pathological conditions. More specifically, the invention concerns methods and compositions for modulating molecular events and outcomes associated with angiogenesis.

### BACKGROUND

The vascular system plays key roles in the maintenance of normal physiological functions. For instance, endothelial cells of the vasculature normally form a cellular barrier that serves to regulate movement of fluid, electrolytes, and proteins into tissues and organs. Although the events that lead to loss of endothelial barrier function is rather complex and not completely understood, the development and maintenance of integrity of the vasculature has been studied extensively. See, e.g., Kagsbrun & Moses, Chemistry & Biology (1999), 6:R217-R224; Ferrara, Endocrine Reviews (2004), 25(4):581-611.

Nonetheless, what is clear is that perturbations of the integrity of the vascular system are implicated in the etiology of multiple diseases and pathological conditions, ranging from cancer to inflammatory diseases such as autoimmune disorders. See, e.g., US Patent Appl. Pub. No. 2004/0167198; Cines et al., Blood (1998), 91(10):3527-3561; Kumar et al., Curr. Opin. In Nephrology and Hypertension (2005), 14:33-37; Carmeliet & Collen Am. J. Physiol. (1997), 273:H2091-H2104. On the other hand, it should be noted that, under certain physiological settings, ability to develop and/or maintain a compromised state of vasculature is desirable, and could, if deficient, itself lead to pathological problems or sub-optimal therapeutic outcomes.

Inflammation associated with the vasculature has been identified as a downstream event of extracellular angiogenic stimuli that is often associated with perturbation of vascular integrity. Kirkpatrick et al., Int. J. Microcirc. (1997), 17:231-240. For example, there is increasing evidence that the inflammatory process may play a significant role in the pathophysiology of various vascular-related disorders, such as hypertension. See, e.g., Touyz, Cur. Opin. Nephrology and Hypertension (2005), 14:125-131; Kobayashi & Lin, Frontiers in Bioscience (2005), 10:666-674; Bacon, Curr. Opin, Rheumatol.(Jan 2005), 17:49-55; Paleolog, J. Clin. Pathol.: mol. Pathol. (1997), 50-225-233; Mullenix et al., Ann. Vasc. Surg. (2005), 19:130-138. As noted in Touyz, events indicative of inflammation are observed in the vasculature, for example increased expression of surface adhesion molecues and their ligands, inflammatory cell infiltration, cytokine production, chemokine release, oxidative stree and activation of innate immunity. Endothelial cell perturbation may also be reflected by the release of von Willebrand factor and E-selectin, which is expressed exclusively by endothelial cells. Kuenen et al., Arterioscler. Thromb. Vasc. Biol. (2002), 22:1500-1505. Persistent and/or unregulated inflammation can result in tissue/vascular damage. The inflammatory response typically involves alterations in vascular permeability, leukocyte extravasation (adhesion, transmigration and chemotaxis) and tissue repair. Touyz, *supra*.

Unfortunately, to date, information regarding intracellular molecules that mediate signaling between extracellular angiogenic stimuli and related downstream events (such as endothelial cell-associated inflammation) has been lacking. This has hampered efforts to develop therapeutic strategies that would permit fine-tuning of the angiogenic process to minimize perturbations of vascular integrity resulting from undesired inflammation, or to induce perturbation of vascular integrity where desired. Therefore, there is a real need to identify "intermediate" molecular targets and pathways that could be the focus of novel therapeutic intervention. The invention described herein meets this need and provides other benefits.

All references cited herein, including patent applications and publications, are incorporated by reference in their entirety.

### DISCLOSURE OF THE INVENTION

The invention is based at least in part on the discovery that a member of the Down syndrome critical region protein family is a critical intermediate molecular link between extracellular signaling by angiogenic factors and intracellular regulation of downstream events that modulate vascular integrity. Specifically, Down syndrome critical region 1 (hereinafter "DSCR1") is shown herein to be an intracellular molecule that is induced in activated endothelial cells following stimulation by angiogenic regulators such as vascular endothelial growth factor (VEGF), whereby induction of DSCR1 in turn modulates expression of genes associated with vascular/endothelial cell inflammation. As described above, inflammation associated with the vasculature has been implicated in a multitude of disorders. Notably, DSCR1 is a particularly advantageous target for therapeutic modulation, since it acts via a negative regulatory feedback loop in mediating between extracellular angiogenic signals and intracellular regulation of vascular inflammation. Without being bound by theory, depending on the cellular/physiological context, DSCR1, in its capacity as a component of a negative regulatory feedback loop, is expected to be sensitive to both positive or negative modulation to alter the molecular and signaling balance normally associated with a regulatory feedback loop. Consistent with this, as shown herein, both increased and decreased DSCR1 activity in endothelial cells are empirically demonstrated to result in similar modulation of cell survival outcomes. Thus, the data disclosed herein reveal an important molecular target/pathway which, when interfered with, results in modulation of one of the key downstream events associated with angiogenic signaling, namely inflammation associated with the vasculature. Under certain circumstances, such inflammation, if left unchecked, can lead to undesirable pathological effects. Yet in other instances, enhancement of such inflammation may be desirable. The invention provides methods, compositions, kits and articles of manufacture useful for regulating such inflammation, by interfering with DSCR1 function/activity so as to modulate vascular inflammation and, therefore, vascular integrity.

In one aspect, the invention provides a DSCR1 modulator that modulates DSCR1 activity in endothelial cells. In one embodiment, the DSCR1 modulator modulates DSCR1 activity induced by signaling through VEGF receptor 2 (VEGFR-2). A DSCR1 modulator of the invention may modulate one or more aspects of the DSCR1-calcineurin A (CnA) pathway. For example, in one embodiment, a DSCR1 modulator of the invention modulates one or more cellular events associated with NFAT activity, including, for example, NFAT phosphorylation, NFAT nuclear translocation or NFAT transcriptional activity (e.g., NFAT's ability to mediate regulation of gene expression by CnA).

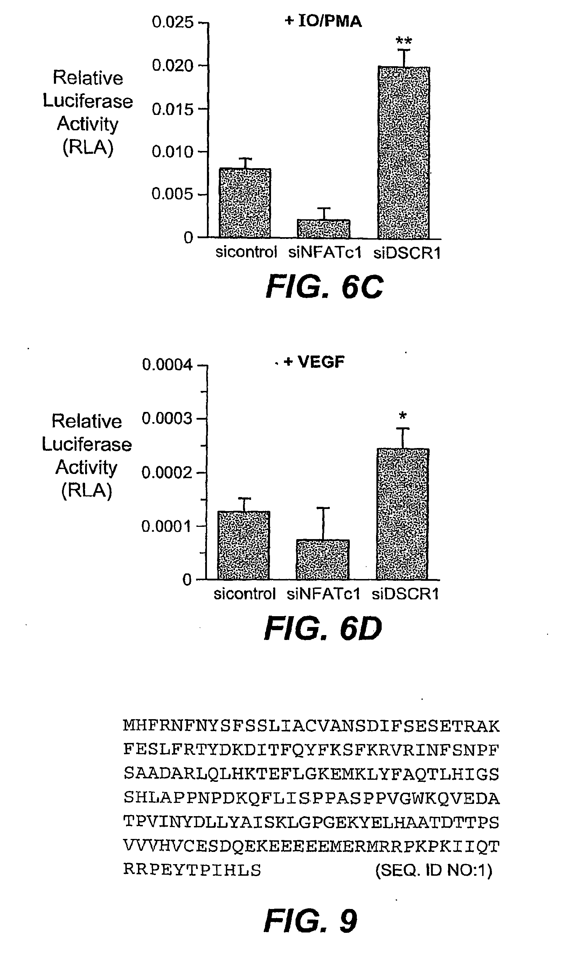

A DSCR1 modulator of the invention can be provided in any form suitable for clinical use. For example, a modulator of the invention can be provided and/or administered in the form of a nucleic acid or polypeptide. Accordingly, in one embodiment, a DSCR1 modulator of the invention comprises a nucleic acid that encodes a polypeptide capable of antagonizing gene expression induced by CnA. For example, the polypeptide may comprise a peptide comprising at least a portion of DSCR1, wherein the peptide is capable of interacting with CnA. In one embodiment, the polypeptide comprises a portion, but not the complete mature DSCR1 protein. In one embodiment, the polypeptide comprises full length mature DSCR1 protein. In one embodiment, the polypeptide comprises amino acid from about position 96 to about 125, or about position 108 to about 122 of human DSCR1, wherein amino acid positions refer to the sequence depicted in SEQ ID NO:1 (Figure 9). In one embodiment, the polypeptide comprises a sequence having at least 50%, 60%, 70%, 80%, 90%, 95%, 98% sequence identity or similarity with the sequence of human DSCR1 from about position 96 to about 125, or about 108 to about 122 of human DSCR1, wherein amino acid positions refer to the sequence depicted in SEQ ID NO:1 (Figure 9), provided the polypeptide retains an ability to antagonize gene expression induced by CnA. In one embodiment, the polypeptide comprises at least a portion of the serine-proline (SP) sequence and at least a portion of the N and/or C-terminal calcineurin binding domain of human DSCR1, wherein the polypeptide is capable of binding to CnA to inhibit CnA-regulated gene expression. In one embodiment, the polypeptide comprises (i) the sequence of human DSCR1 from about position 96 to about 125, or about 108 to about 122 of human DSCR1, wherein amino acid positions refer to the sequence depicted in SEQ ID NO:1 (Figure 9); (ii) at least a portion of the serine-proline (SP) sequence; and (iii) at least a portion of the N and/or C-terminal calcineurin binding domain of human DSCR1, wherein the polypeptide is capable of binding to CnA to inhibit CnA-regulated gene expression.

In one aspect, the invention provides a DSCR1 modulator capable of antagonizing DSCR1 activity. Accordingly, in one embodiment, a DSCR1 modulator of the invention comprises a nucleic acid that when present in an endothelial cell (e.g., an activated endothelial cell) inhibits activity (including but not limited to gene expression) of DSCR1 in the cell. In one embodiment, the nucleic acid comprises an antisense oligonucleotide. In another embodiment, the nucleic acid comprises an inhibitory/interfering RNA. In one embodiment, the inhibitory/interfering RNA is a small inhibitory/interfering RNA (siRNA). For example, a DSCR1 modulator comprising siRNA may comprise one or more sequence selected from the group consisting of GCUCAGACCUUACACAUAG, GGACAUCACCUUUCAGUAU, GAAAGAAUGAGGAGACCUA and GACAUCACCUUUCAGUAUU. In another example, a DSCR1 modulator comprising siRNA may comprise one or more sequence selected from the group consisting of AACGUAUGACAAGGACAUCAC,
AAGGACAUCACCUUUCAGUAU,AAGUUAUAUUUUGCUCAGACCand
AAGAUGCGACCCCAGUCAUAA. In one embodiment, a DSCR1 modulator comprising siRNA comprises one or more sequence selected from the group consisting of
GCUCAGACCUUACACAUAG,GGACAUCACCUUUCAGUAU,
GAAAGAAUGAGGAGACCUA, GACAUCACCUUUCAGUAUU,
AACGUAUGACAAGGACAUCAC,AAGGACAUCACCUUUCAGUAU,
AAGUUAUAUUUUGCUCAGACC and AAGAUGCGACCCCAGUCAUAA.

In one embodiment, the nucleic acid comprises an aptamer.

In one embodiment, a DSCR1 modulator of the invention comprises an antibody. In one embodiment, the antibody is administered such that it enters a target endothelial cell to inhibit DSCR1 in the cell, i.e., the antibody is internalized. In another embodiment, the antibody is encoded by a nucleic acid that is introduced (e.g., transfected) into a target endothelial cell.

In one aspect, the invention provides methods for treating a variety of disorders associated with inflammation associated with endothelial cells. For example, in one aspect, the invention provides a method of treating a disorder associated with abnormal vascular inflammation, said method comprising administering to a subject an effective amount of a DSCR1 modulator of the invention, thereby treating the disorder. In one embodiment, the modulator increases inflammation associated with the vasculature. In one embodiment, the modulator inhibits inflammation associated with the vasculature.

In one aspect, the invention provides a method of treating an immunological disorder associated with abnormal vascular inflammation, said method comprising administering to a subject an effective amount of a DSCR1 modulator of the invention, thereby treating the disorder. In one embodiment, the modulator increases inflammation associated with the vasculature. In one embodiment, the modulator inhibits inflammation associated with the vasculature.

In yet another aspect, the invention provides a method of treating a disorder associated with perturbation of vascular integrity, said method comprising administering to a subject an effective amount of a DSCR1 modulator of the invention, thereby treating the disorder. In one embodiment, the perturbation of vascular integrity is associated with vascular/endothelial cell-related inflammation, for example where there is an undesirably high or low amount of vascular/endothelial cell-related inflammation. In one embodiment, the modulator increases inflammation associated with the vasculature. In one embodiment, the modulator inhibits inflammation associated with the vasculature.

In one aspect, the invention provides a method of ameliorating side-effects associated with anti-inflammatory therapy comprising administering to a subject a DSCR1 modulator of the invention, in conjunction with the anti-inflammatory therapy, thereby ameliorating said side-effects. In one embodiment, the DSCR1 modulator inhibits DSCR1 activity in an endothelial cell. In one embodiment, the anti-inflammatory therapy comprises an agent that suppresses inflammation, for example the agent could inhibit immune cell activity. In one embodiment, the agent is of the cyclosporine class of inflammation suppressors, such as cyclosporine A (CsA). In one embodiment, the modulator increases inflammation associated with the vasculature. In one embodiment, the modulator inhibits inflammation associated with the vasculature.

In yet another aspect, the invention provides a method of treating a disorder associated with undesirable angiogenesis (such as cancer), said method comprising administering to a subject with the disorder an anti-angiogenic agent and a DSCR1 modulator of the invention.

In another aspect, the invention provides a method of inhibiting tumor growth or growth of a cancer cell, said method comprising administering to the tumor or the cancer cell an effective amount of an anti-cancer agent and an effective amount of a DSCR1 modulator of the invention, wherein the combined effective amounts inhibit tumor or growth of the cancer cell.

In one embodiment, an anti-cancer agent comprises an anti-angiogenic agent, for example a VEGF antagonist. In one embodiment, a VEGF antagonist is an anti-VEGF antibody. In one embodiment, an anti-VEGF antibody comprises an antigen binding sequence of antibody A4.6.1 or affinity matured variants thereof. In one embodiment, the antigen binding sequence comprises at least one, two or three of the hypervariable regions of the heavy chain of antibody A4.6.1 or affinity matured variants thereof. In one embodiment, the antigen binding sequence comprises at least one, two or three of the hypervariable regions of the light chain of antibody A4.6.1 or affinity matured variants thereof. In one embodiment, the antigen binding sequence comprises a humanized form of the heavy chain variable domain of antibody A4.6.1 or affinity matured variants thereof. In one embodiment, the antigen binding sequence comprises a humanized form of the light chain variable domain of antibody A4.6.1 or affinity matured variants thereof. Antibody A4.6.1 is an anti-VEGF antibody produced by the hybridoma cell line deposited under ATCC deposit no. HB 10709 (American Type Culture Collection, Manassas, VA). In one embodiment, the anti-VEGF antibody is bevacizumab. In one embodiment, the anti-VEGF antibody is an antibody that binds to the same epitope of human VEGF as bevacizumab. In one embodiment, the anti-VEGF antibody is an antibody that competes with bevacizumab for binding to human VEGF. In one embodiment, the anti-VEGF antibody binds to a receptor of VEGF, for example VEGFR2. In one embodiment of these methods, a DSCR1 modulator of the invention is administered to a subject in conjunction with an anti-cancer agent such as a chemotherapeutic agent. In one embodiment, the DSCR1 modulator inhibits side effects associated with perturbation of vascular integrity. For example, the perturbation of vascular integrity may be associated with thrombolysis.

In one aspect, the invention provides a method of treating a disorder by enhancing angiogenesis, said method comprising administering to a subject with the disorder an effective amount of an agent that induces angiogenesis and an effective amount of a DSCR1 modulator, whereby the disorder is treated. In one embodiment, the agent that induces angiogenesis comprises a pro-angiogenic substance, for example one or more of the angiogenic factors disclosed herein. In one embodiment, the angiogenic factor is VEGF. In one embodiment, the DSCR1 modulator inhibits perturbation of vascular integrity. A disorder that can be treated by this method include any of those described herein, including for example a wound, ischemia, a cardiovascular disorder, atherosclerosis, vasculitis, etc.

In one aspect, the invention provides a method of modulating survival of an endothelial cell comprising contacting the cell with a DSCR1 modulator. In one embodiment, the endothelial cell is activated. In one embodiment, the endothelial cell is transfected with an expression vector encoding a DSCR1 antisense sequence. In one embodiment, the endothelial cell is transfected with an expression vector encoding a DSCR1 sense sequence. In one embodiment, the DSCR1 modulator comprises an inhibitory/interfering RNA, which in one embodiment is a small interfering RNA (siRNA).

In methods of the invention wherein a DSCR1 modulator of the invention is administered in conjunction with another therapeutic agent, the timing of administration of DSCR1 modulator relative to the timing of administration of said another therapeutic agent would be any that is deemed empirically and/or clinically to be therapeutically beneficial. For example, in one embodiment, the DSCR1 modulator is administered prior to administration of said another therapeutic agent. In one embodiment, the DSCR1 modulator is administered subsequent to administration of said another therapeutic agent. In another embodiment, the DSCR1 modulator is administered concurrently with administration of said another therapeutic agent. In one embodiment, said another therapeutic agent has anti-inflammatory activity. In one embodiment, the two agents are administered as separate compositions. In one embodiment, the two agents are administered as a single composition.

In one aspect, the invention provides a method of identifying a subject at risk of developing side effects associated with treatment of a disorder comprising modulation of angiogenesis, said method comprising measuring amount of DSCR1 expression or activity in a tissue or cell and comparing the amount to that of a normal counterpart tissue or cell, wherein a difference in amount indicates a risk of developing said side effects. Generally, the side effects are due to perturbation of vascular integrity associated with inflammation. The method of identifying can be practiced at any convenient and/or beneficial timepoint during the clinical intervention process. For example, it can occur before, during or after administration of a therapeutic agent to treat the disorder in question.

In one aspect, the invention provides a method of identifying a DSCR1 modulator (such as a molecule useful for modulating perturbations of vascular integrity/vascular inflammation), the method comprising contacting a candidate substance with DSCR1 (or functional variant thereof as defined hereinbelow), and determining amount of the DSCR1 (or functional variant thereof) in the presence and absence of the candidate substance, wherein - differential amounts of the DSCR1 (or functional variant thereof) in the presence and absence of the candidate substance indicates that the candidate substance is a DSCR1 modulator. DSCR1 amount can be determined by any suitable qualitative or quantitative measurement known in the art. For example, DSCR1 amount may be indicated by level of RNA or protein. In another example, DSCR1 amount is indicated by DSCR1 activity (e.g., inhibition of CnA). Methods of the invention can be used either to identify DSCR1 antagonists or agonists. For example, in one embodiment, DSCR1 amount is lower in the presence of the candidate substance. In another embodiment, DSCR1 amount is higher in the presence of the candidate substance. A modulator identified by a method of the invention can have any of the characteristics required for use in methods of the invention. For example, a candidate can be selected that is capable of modulating vascular integrity and/or inflammation associated with endothelial cells. Alternatively, a candidate substance can be additionally evaluated to select a substance that lacks certain undesirable characteristics. For example, a candidate substance can be selected that does not substantially modulate endothelial cell proliferation.

As noted above, DSCR1 modulators and methods of the invention are useful in treating a variety of disorders suspected or known to be associated with improper regulation of vascular integrity. For example, these disorders include, but are not limited to, those that are in the vascular (such as cardiovascular), endothelial, angiogenic (such as tumor/cancer) or immunological (such as autoimmune) categories of disorders. Nonetheless, in some embodiments, these disorders are not cardiovascular or immunological. In some embodiments, these disorders are not endothelial or angiogenic. Yet in other embodiments, the disorders are associated with tissue inflammation (acute or chronic), for example autoimmune disorders. It should also be noted that some diseases may have characteristics that overlap between two or more categories of disorders.

In general, the invention provides modulator molecules and methods that are useful for treatment of immune-mediated disorders. In one embodiment, the invention relates to treatment of autoimmune disorders, such as those indicated herein. In one embodiment, a disorder relates to graft rejection or graft versus host disease.

In some embodiments of methods of the invention wherein a treatment method comprises promoting angiogenesis /neovascularization in order to treat a disorder, the disorder may be, but is not limited to, e.g., vascular trauma, wounds, lacerations, incisions, bums, ulcers (e.g., diabetic ulcers, pressure ulcers, haemophiliac ulcers, varicose ulcers), tissue growth, weight gain, peripheral arterial disease, induction of labor, hair growth, epidermolysis bullosa, retinal atrophy, bone fractures, bone spinal fusions, meniscal tears, etc.

Where clinically appropriate or desired, methods of the invention can further comprise additional treatment steps. For example, in one embodiment, a method further comprises a step wherein a targeted cell and/or tissue (e.g., a cancer cell) is exposed to radiation treatment or a chemotherapeutic agent.

In one aspect, the invention provides compositions comprising one or more DSCR1 modulators of the invention and a carrier. In one embodiment, the carrier is pharmaceutically acceptable.

In one aspect, the invention provides nucleic acids encoding a DSCR1 modulator of the invention. In one embodiment, a nucleic acid of the invention encodes a DSCR1 modulator which is or comprises a polypeptide (e.g., an oligopeptide). In one embodiment, a nucleic acid of the invention encodes a DSCR1 modulator which is or comprises an antibody or fragment thereof. In one embodiment, a nucleic acid of the invention encodes a nucleic acid sequence that inhibits DSCR1 expression/activity (e.g., DSCR1 gene transcription or translation of DSCR1 protein).

In one aspect, the invention provides vectors comprising a nucleic acid of the invention.

In one aspect, the invention provides host cells comprising a nucleic acid or a vector of the invention. A vector can be of any type, for example a recombinant vector such as an expression vector. Any of a variety of host cells can be used. In one embodiment, a host cell is a prokaryotic cell, for example, *E*. *coli*. In one embodiment, a host cell is a eukaryotic cell, for example a mammalian cell such as Chinese Hamster Ovary (CHO) cell. In one embodiment, a nucleic acid or vector of the invention is expressed in an endothelial cell.

In one aspect, the invention provides methods for making a DSCR1 modulator of the invention. For example, the invention provides a method of making a DSCR1 modulator which is or comprises an antibody (or fragment thereof), said method comprising expressing in a suitable host cell a recombinant vector of the invention encoding said antibody (or fragment thereof), and recovering said antibody (or fragment thereof). In another example, the invention provides a method of making a DSCR1 modulator which is or comprises a polypeptide (such as an oligopeptide), said method comprising expressing in a suitable host cell a recombinant vector of the invention encoding said polypeptide (such as an oligopeptide), and recovering said polypeptide (such as an oligopeptide).

In one aspect, the invention provides an article of manufacture comprising a container; and a composition contained within the container, wherein the composition comprises one or more DSCR1 modulators of the invention. In one embodiment, the composition comprises a nucleic acid of the invention. In one embodiment, a composition comprising a DSCR1 modulator further comprises a carrier, which in some embodiments is pharmaceutically acceptable. In one embodiment, an article of manufacture of the invention further comprises instructions for administering the composition (e.g., the DSCR1 modulator) to treat a disorder indicated herein.

In one aspect, the invention provides a kit comprising a first container comprising a composition comprising one or more DSCR1 modulators of the invention; and a second container comprising a buffer. In one embodiment, the buffer is pharmaceutically acceptable. In one embodiment, a composition comprising a DSCR1 modulator further comprises a carrier, which in some embodiments is pharmaceutically acceptable. In one embodiment, a kit further comprises instructions for administering the composition (e.g., the DSCR1 modulator) to treat a disorder indicated herein.

Some additional embodiments of the invention are described in the following paragraphs.
1. A DSCR1 modulator that modulates DSCR1 activity in endothelial cells.
2. The DSCR1 modulator of para 1, wherein the modulator modulates DSCR1 activity induced by signaling through VEGF receptor 2 (VEGFR-2).
3. The DSCR1 modulator of any of the preceding paras, wherein the modulator modulates the DSCR1-calcineurin A (CnA) pathway.
4. The DSCR1 modulator of any of the preceding paras , wherein the modulator modulates a cellular event associated with NFAT activity.
5. The DSCR1 modulator of para 4, wherein the cellular event is NFAT phosphorylation, NFAT nuclear translocation or NFAT transcriptional activity.
6. The DSCR1 modulator of any of the preceding paras , wherein the modulator comprises a nucleic acid that encodes a polypeptide capable of antagonizing gene expression induced by CnA.
7. The DSCR1 modulator of para : 6, wherein the polypeptide comprises a peptide comprising at least a portion of DSCR1, wherein the peptide is capable of interacting with CnA.
8. The DSCR1 modulator of para 6 or 7, wherein the polypeptide comprises amino acid from about position 96 to about 125, or about position 108 to 122 of human DSCR1.
9. The DSCR1 modulator of para 1 or 2, wherein the modulator is capable of antagonizing DSCR1 function.
10. The DSCR1 modulator of para 9, wherein the modulator comprises a nucleic acid that when present in an endothelial cell inhibits activity of DSCR1 in the cell.
11. The DSCR1 modulator of para 10, wherein the nucleic acid comprises an antisense oligonucleotide.
12. The DSCR1 modulator of para 10, wherein the nucleic acid comprises an inhibitory/interfering RNA.
13. The DSCR1 modulator of para 12, wherein the inhibitory/interfering RNA is a small inhibitory/interfering RNA (siRNA).
14. The DSCR1 modulator of para 12 or 13, wherein the nucleicacid comprises one or more sequence selected from the group consisting of
   GCUCAGACCUUACACAUAG, GGACAUCACCUUUCAGUAU,
   GAAAGAAUGAGGAGACCUA, GACAUCACCUUUCAGUAUU,
   AACGUAUGACAAGGACAUCAC,AAGGACAUCACCUUUCAGUAU,
   AAGUUAUAUUUUGCUCAGACCandAAGAUGCGACCCCAGUCAUAA.
15. The DSCR1 modulator of para 10, wherein the nucleic acid comprises an aptamer.
16. A method of treating a disorder associated with abnormal vascular inflammation, said method comprising administering to a subject an effective amount of a DSCR1 modulator of any of paras 1-15, thereby treating the disorder.
17. The method of para 16, wherein the modulator increases inflammation associated with the vasculature.
18. The method of para 16, wherein the modulator inhibits inflammation associated with the vasculature.
19. A method of treating an immunological disorder associated with abnormal vascular inflammation, said method comprising administering to a subject an effective amount of a DSCR1 modulator of any of paras 1-15, thereby treating the disorder.
20. The method of para 19, wherein the modulator increases inflammation associated with the vasculature.
21. The method of para 20, wherein the modulator inhibits inflammation associated with the vasculature.
22. A method of treating a disorder associated with perturbation of vascular integrity, said method comprising administering to a subject an effective amount of a DSCR1 modulator of any of paras 1-15, thereby treating the disorder.
23. The method of para 22, wherein the perturbation of vascular integrity is associated with vascular/endothelial cell-related inflammation.
24. The method of para 23, wherein the modulator increases inflammation associated with the vasculature.
25. The method of para 23, wherein the modulator inhibits inflammation associated with the vasculature.
26. A method of ameliorating side-effects associated with anti-inflammatory therapy comprising administering to a subject a DSCR1 modulator of any of paras 1-15, in conjunction with the anti-inflammatory therapy, thereby ameliorating said side-effects.
27. The method of para 26, wherein the DSCR1 modulator inhibits DSCR1 activity in an endothelial cell.
28. The method of para 26 or 27, wherein the anti-inflammatory therapy comprises an agent that suppresses inflammation.
29. The method of para 26, wherein the modulator increases inflammation associated with the vasculature.
30. The method of para 26, wherein the modulator inhibits inflammation associated with the vasculature.
31. A method of treating a disorder associated with undesirable angiogenesis, said method comprising administering to a subject with the disorder an anti-angiogenic agent and a DSCR1 modulator of any of paras 1-15.
32. A method of inhibiting tumor growth or growth of a cancer cell, said method comprising administering to the tumor or the cancer cell an effective amount of an anti-cancer agent and an effective amount of a DSCR1 modulator of any of paras 1-15, wherein the combined effective amounts inhibit tumor or growth of the cancer cell.
33. The method of para 32, wherein the anti-cancer agent comprises an anti-angiogenic agent.
34. The method of para 33, wherein the anti-angiogenic agent comprises a VEGF antagonist.
35. The method of para 34, wherein the VEGF antagonist is an anti-VEGF antibody.

### BRIEF DESCRIPTION OF THE DRAWINGS

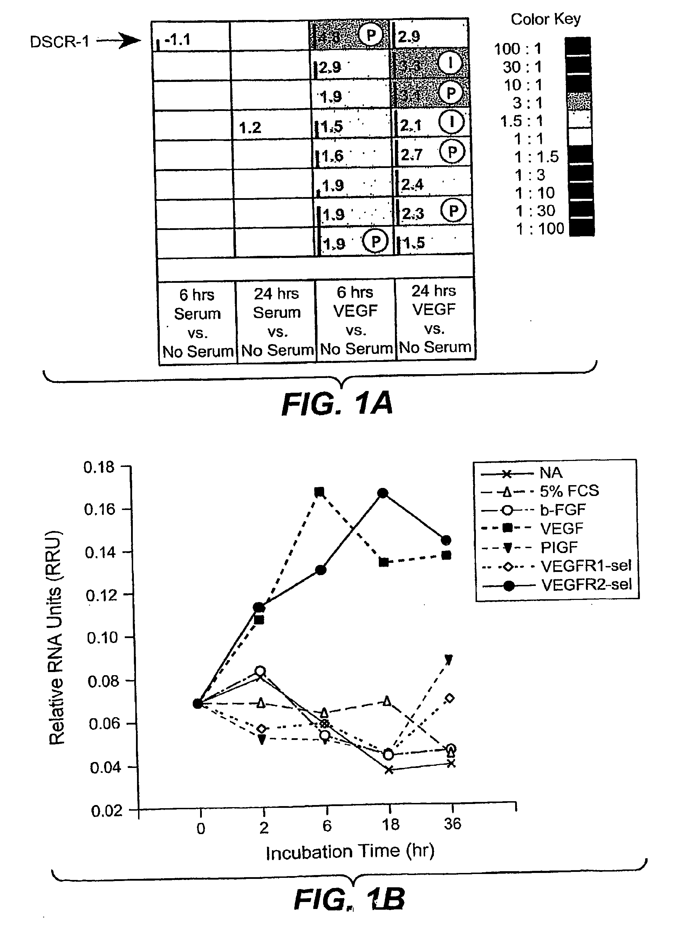

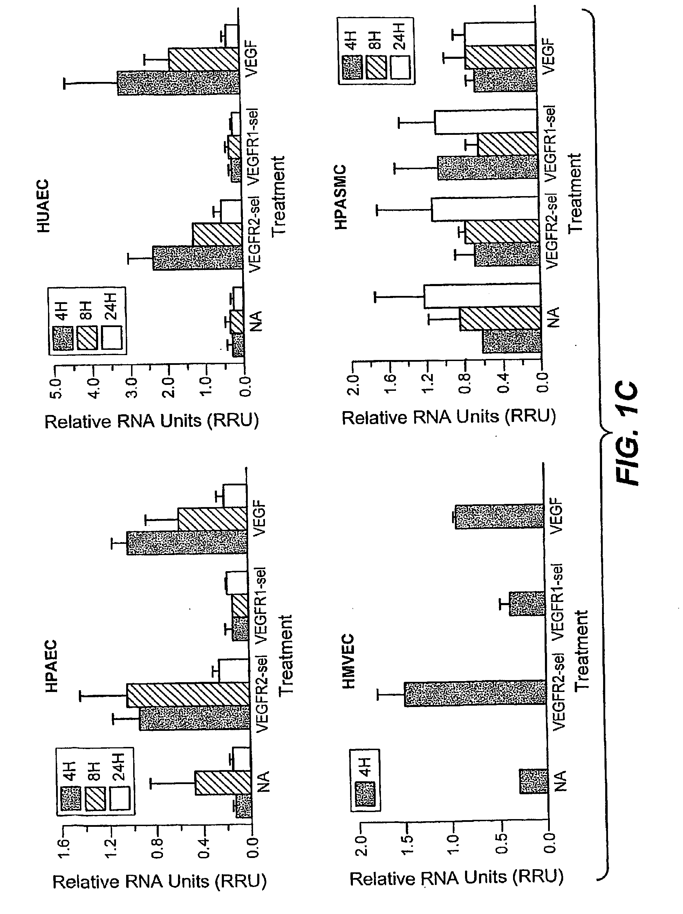

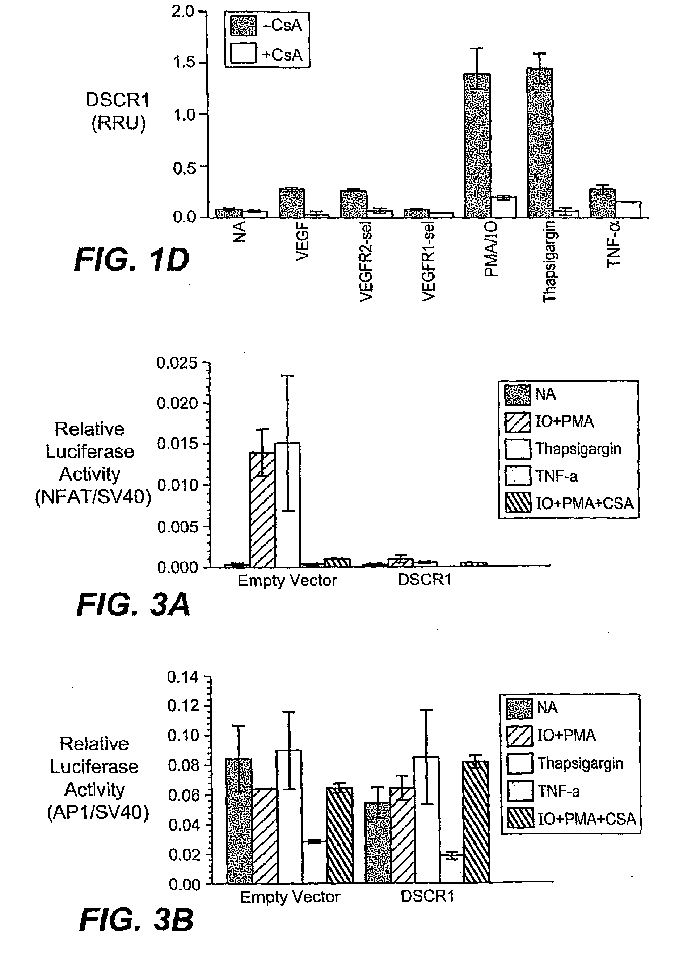

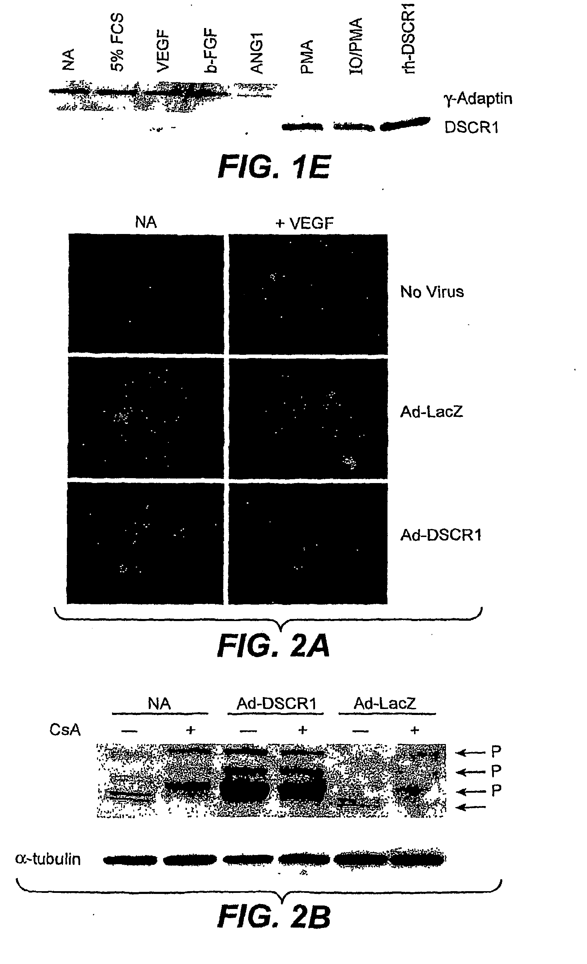

**Figure 1****.** Gene expression analysis using GeneCall technology and RT-PCR. (A) Primary HUVECs were exposed for 0, 6, or 24 hours to either serum-free media or serum-free media complemented with VEGF or 5% FCS, respectively. Endothelial cells were lysed and total RNA was isolated and analyzed for gene expression by using GeneCalling. Each column represents the output of a series of binary comparisons that have been compared to each other. However, genes marked with "P" (P = poisoning) or "I" (I = Isolation, cloning, sequencing, and poisoning) were confirmed. Selected genes shown were found induced by VEGF at 6 and 24 hours, but not by stimulation by 5% FCS; the fold stimulation by VEGF is indicated by a number in each box. Gray boxes indicate that no change in expression was observed for the genes in the binary comparisons for serum at 6 and 24 hours versus no serum. Vertical black bars in the enlarged thumbnail indicate the confidence level of the gene call. (B) DSCR1 expression analysis by real-time PCR.²¹ HUVECs were kept in serum-free conditions and stimulated with either 5% FCS, b-FGF (10 ng/mL), VEGF (30 ng/mL), PIGF (100 ng/mL), VEGFR1-sel (100 ng/mL), or VEGFR2-sel (30 ng/mL) during the indicated amount of time. The mutant proteins used were VEGFR2-sel (KDR-selective): VEGF D63S/G65M/L66R and Flt-selective VEGF I43A/I46A/Q79A/I83.²⁵ (C) DSCR1 expression analysis by real-time PCR. HPAECs, HDMECs, and HUAECs were incubated in presence of 0.5% FCS (NA), or in presence of 0.5% FCS and VEGF (30 ng/mL) or VEGF mutant forms, binding to either VEGFR1 (VEGFR1-sel, 100 ng/mL) or VEGFR2 (VEGFR2-sel, 30 ng/mL)²⁶ for 4( ■,) 8 ( 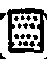 ), and 24 (□) hours. As control cells, HUPASMCs were used, which do not express significant levels of VEGF receptors (data not shown). Data shown represent the average ± SD of triplicate samples of one representative of total 3 independent experiments. Relative RNA units (RRUs) were calculated as described by Gerber et al³ by using cyclophylin as reference gene. (D) DSCR1 expression analysis by real-time PCR. HUVECs were incubated for 2 hours in presence of CsA (1 µM) prior stimulation with VEGF (30 ng/mL), VEGFR2-sel (30 ng/mL), VEGFR1-sel (100 ng/mL), PMA (200 ng/mL), and IO (5 µM), thapsigargin (50 nM) or TNF-α (10 ng/mL) for 5.5 hours, as indicated. Data shown represent the average ± SD of duplicate samples run in parallel of one representative of total 2 independent experiments. (E) Western blot analysis of whole cell extracts of HUVECs grown in 0% serum, 0.5% BSA and 5% FCS, hVEGF (30 ng/mL), b-FGF (10 ng/mL), TNF-α (10 ng/mL) and PMA or ionomycine (IO) for 5 hours as indicated. As control, 100 ng recombinant DSCR1 protein was included.
**Figure 2****.** Cellular localization of DSCR1 and NFATc1 phosphorylation. (A) IHC analysis of HUVECs transduced with Ad-DSCR1-FLAG or control Ad-LacZ (MOI = 100) and exposed to VEGF (30 ng/mL). After 20 minutes of stimulation by VEGF, cells were fixed and cellular localization of NFATc1 was analyzed. (B) Western blot analysis of NFATc1 phosphorylation. HUVECs were transduced with the indicated adenoviral vectors (MOI = 100) or exposed to transduction media only (NA). Two days after transduction, CsA (1 µM) was added to cells and 2 hours later, cells were stimulated with PMA (200 ng/mL)/IO (5 µM) for 4 hours. Total cell extracts were subjected to Western blotting analysis using an anti-NFATc1 antibody, which recognizes NFATc1 independently of the phosphorylation status. Data shown are from 1 representative of total 3 experiments.
**Figure 3****.** DSCR1 interferes with transcriptional activity of NFAT in activated endothelial cells. (A) Luciferase reporter gene analysis of HUVECs transfected with a mixture including CMV-driven expression vectors encoding a C-terminally epitope tagged version of DSCR1 (DSCR1-FLAG) or equal amounts of an empty vector. Luciferase reporter constructs contained either 3 NFAT-binding sites (NFAT-Luc) or 3 copies of an AP1 (B) binding sites, respectively. Forty-eight hours after transfection, cells were stimulated with PMA (200 ng/mL), thapsigargin (50 nM), or IO (5 µM) for 6 hours. Cells were lysed and analyzed for reporter gene expression. Data shown represent lµciferase activity relative to SV40-RL activity. Data represent the means of triplicate samples ± SD of one representative of 4 independent experiments.

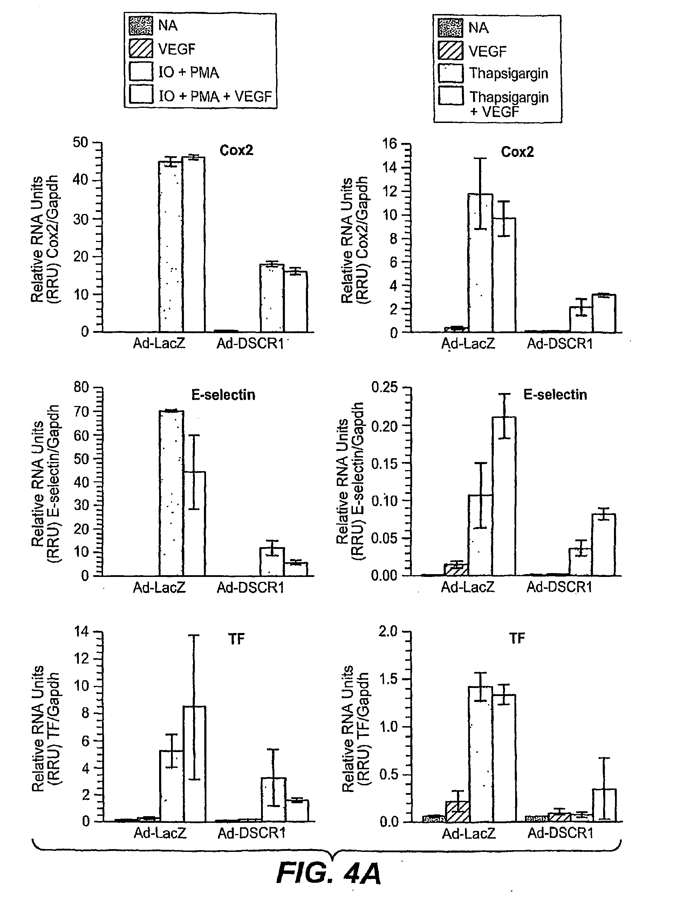

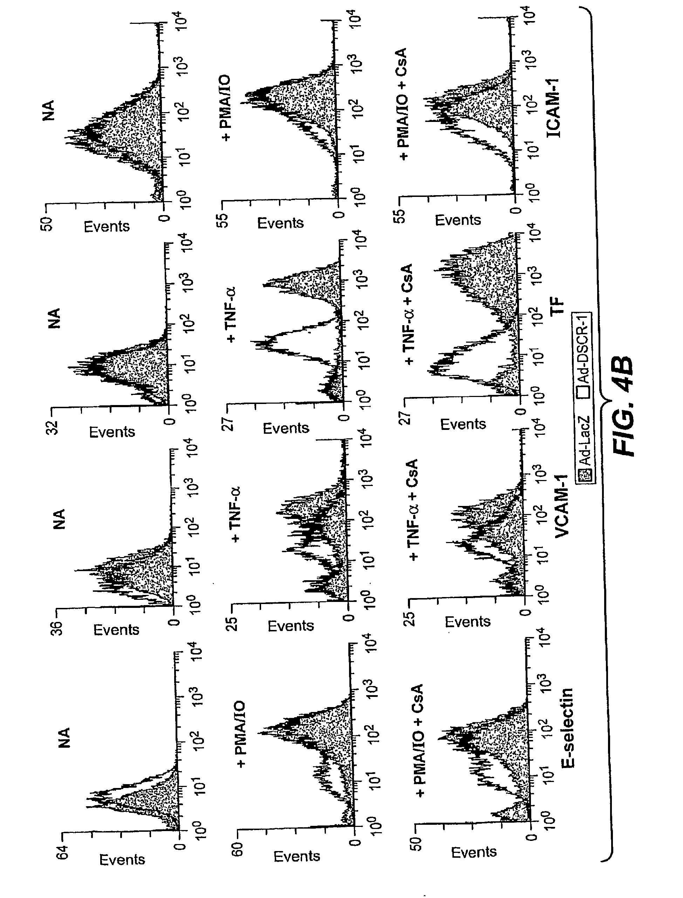

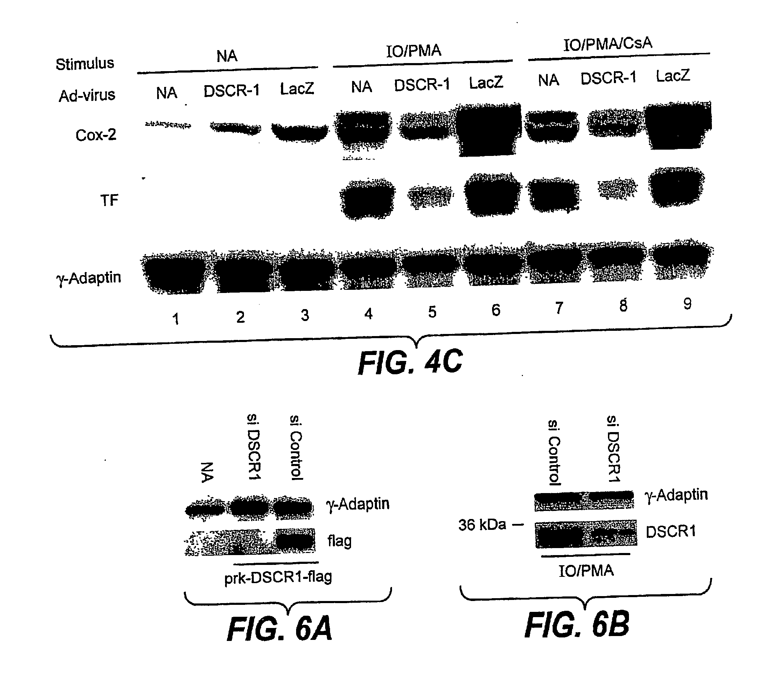

**Figure 4****.** Repression of inflammatory marker gene expression by DSCR1 on activated endothelial cells. (A) Real-time RT-PCR analysis of HUVECs transduced with adenoviral vectors (MOI = 100) encoding for DSCR1 (Ad-DSCR1-FLAG) or control LacZ (Ad-LacZ). Two days after transduction, cells were incubated for 5 to 6 hours with growth medium alone or with growth medium complemented with PMA (200 ng/mL) and IO (5 µM), VEGF (30 ng/mL), thapsigargin (50 nM), or a combination thereof as indicated. Total RNA was isolated and levels for Cox-2 (i), E-selectin (ii), and TF (iii) were analyzed by real-time RT-PCR analysis. Data shown represent the average ± SD of triplicate samples of one representative of total 3 independent experiments. RRUs were calculated as described by Gerber et al³ by using *GAPDH* as reference gene. (B) FACS analysis of transiently transfected endothelial cells stimulated by various compounds. CsA (1 µM) was added to transduced cells 2 hours prior to stimulating cells with either PBS (NA), PMA (200 ng/mL), IO (5 µM), TNF-α (10 ng/mL), or a combination thereof. At 24 hours after stimulation, cells were removed from the culture dish by incubation in 25 mM EDTA (ethylenediaminetetraacetic acid)/PBS for 10 minutes. Cells were analyzed for expression of E-selectin, VCAM-1, TF, and ICAM-1. White areas under the chromatograms represent expression levels on cells transduced with Ad-DSCR1; gray areas represent expression levels on LacZ-transduced cells. Data shown are from one representative of total 3 independent experiments. (C) Western blotting analysis of total cell extracts of HUVECs for Cox-2 and TF, respectively. To control for equal loading, an antibody recognizing 2γ-adaptin was used. Data shown are from one representative experiment of 3 independent experiments.

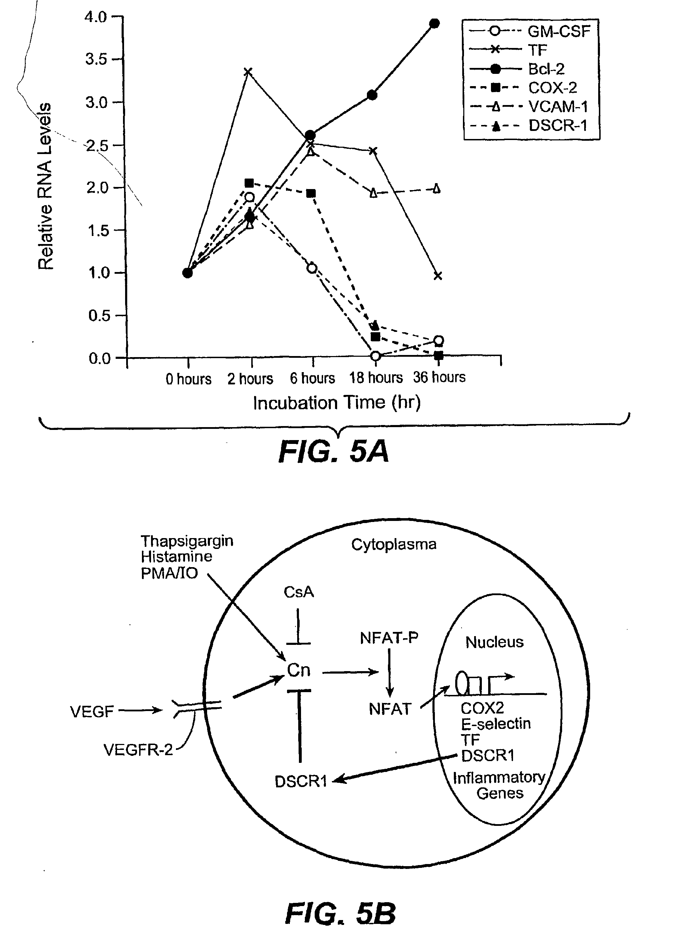

**Figure 5****.** Transient regulation of inflammatory marker genes by VEGF on endothelial cells. (A) Real-time RT-PCR analysis of HUVECs kept in serum-free conditions and stimulated with VEGF (30 ng/mL), or serum-free medium only, during the indicated amount of time. *Taq*Man analysis for expression levels was conducted. The relative expression levels shown were generated by dividing the RRUs of gene expression in presence of VEGF with the non-stimulated levels. (B) Schematic representation of the negative feedback regulatory loop by DSCR1 in activated endothelial cells leading to interference with calcineurin signaling after stimulation by VEGF or compounds activating CnA signaling.
**Figure 6****.** Knock down of endogenous DSCR1 by siRNA enhances NFAT activity in activated endothelial cells. (A) Western blot analysis of HUVECs cotransfected with siRNA and PRKN-DSCR1-Flag expression vector. Whole cell extracts were analyzed using an anti-FLAG antibody. (B) HUVECs transfected with siDSCR1 were analyzed for endogenous levels of DSCR1 by using a polyclonal antiserum detecting human DSCR1. (C) Luciferase reporter gene assay for NFAT activity in cell extracts from HUVECs after transient transfection with NFAT luciferase reporter constructs and the siRNA targeting DSCR1, NFATc1, or control and stimulated with IO/PMA (6 hours) or VEGF (D; 12 hours), respectively. Data shown are from one representative of a total of 3 independent experiments run in triplicate. Error bars = SD. **P* < .01, ***P* < .001, using analysis of variance (ANOVA) by comparing siControl and siDSCR1 groups.

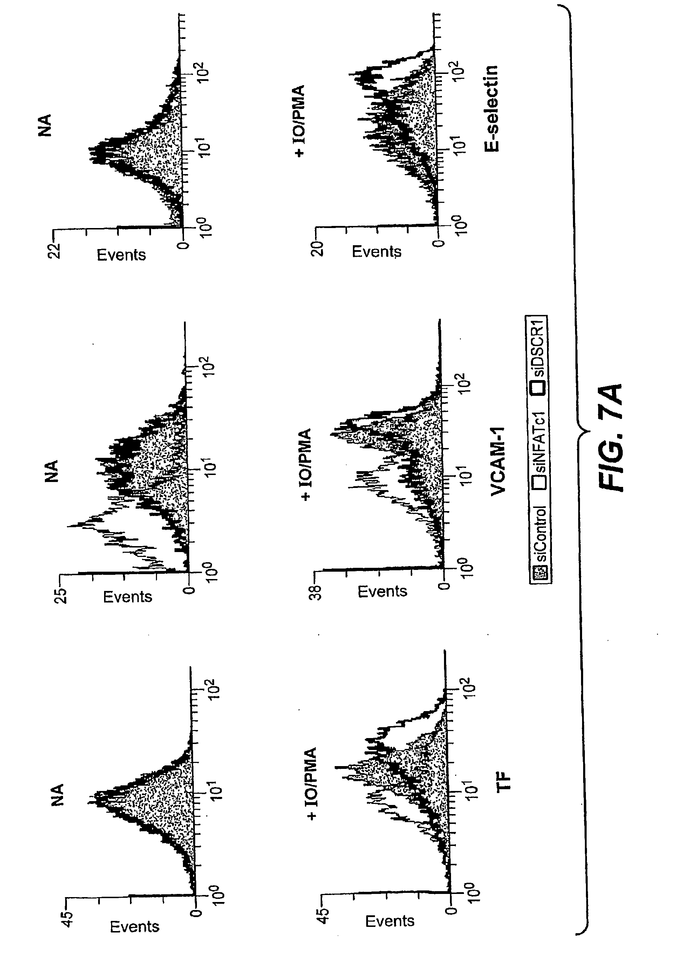

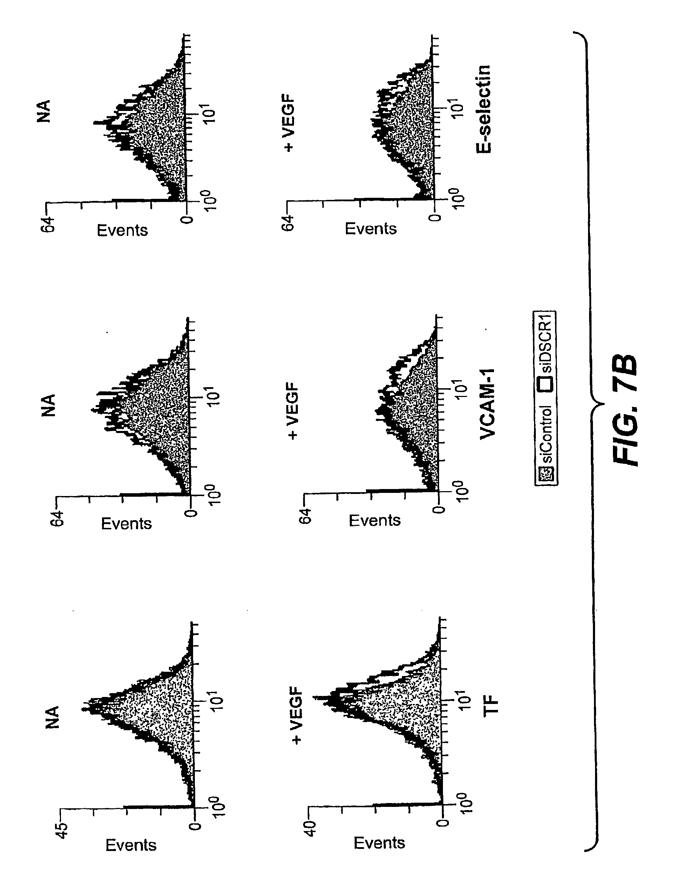

**Figure 7****. Knock down of endogenous DSCR1 induces expression of inflammatory markers on activated endothelial cells.** (A) FACS analysis of HUVECs transiently cotransfected with an expression vector encoding for GFP and siDSCR1, siNFATc1, or siControl siRNA. Cells were analyzed for cell surface expression of TF, VCAM-1, and E-selectin on GFP+ cells 4 hours after stimulation with PMA/IO or control treatment (NA). (B) FACS analysis of HUVECs stimulated with VEGF and analyzed for expression of TF (4 hours), VCAM-1 (20 hours), and E-selectin (20 hours). Data shown correspond to one representative set of 3 independent experiments.

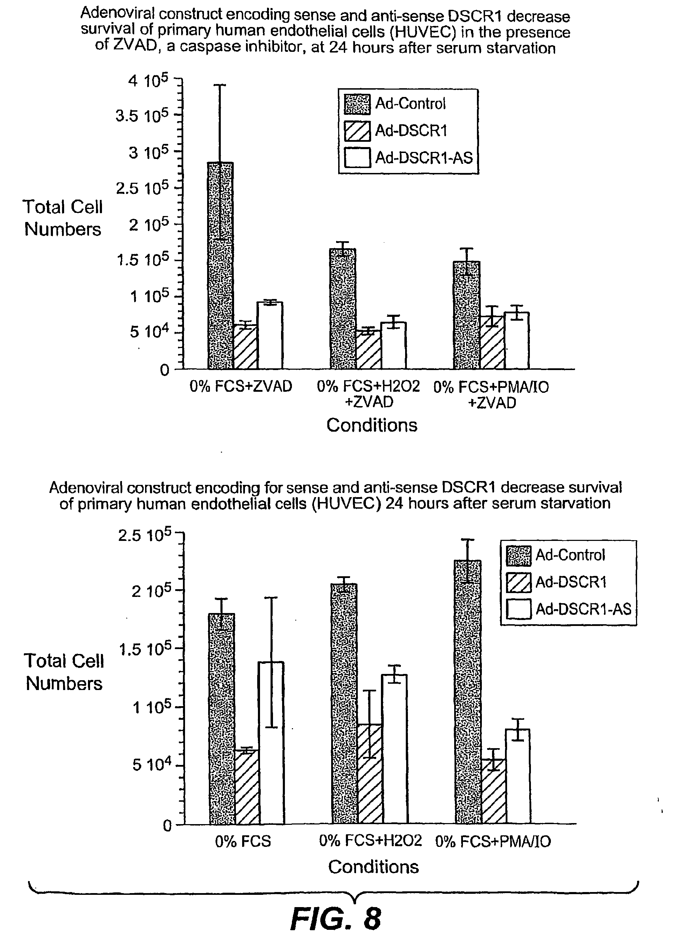

**Figure 8** **Modulation of DSCR1 affects survival of endothelial cells.** Overexpression of DSCR1 by Ad-DSCR1 in endothelial cells exposed to stress conditions, including serum starvation, H₂O₂ exposure, and PMA/IO treatment induces endothelial cell death as did reduction of endogenous DSCR1 by expression of the DSCR1 anti-sense construct.. (A) DSCR1 modulation comprising adenoviral constructs encoding for either sense or anti-sense DSCR1 decreased survival of primary human endothelial cells (HUVEC) 24 hours after serum starvation. (B) DSCR1 modulation comprising adenoviral constructs encoding either sense or anti-sense DSCR1 decreased survival of primary human endothelial cells (HUVEC) in the presence of ZVAD, a caspase inhibitor, at 24 hours after serum starvation.
**Figure 9****.** An illustrative embodiment of an amino acid sequence of human DSCR1 (SEQ ID NO:1).

### MODES FOR CARRYING OUT THE INVENTION

The invention provides methods, compositions, kits and articles of manufacture for treating a variety of disorders by modulating DSCR1 in endothelial cells. Details of these methods, compositions, kits and articles of manufacture are provided herein.

### General Techniques

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., ed., 1994); "A Practical Guide to Molecular Cloning" (Perbal Bernard V., 1988).

### Definitions

As used herein, the term "vascular" or "vasculature", and variations thereof, refers to the system of vessels carrying blood (including lymph fluids) throughout the mammalian body.

"Blood vessel" refers to any of the vessels of the mammalian vascular system, including arteries, arterioles, capillaries, venules, veins, sinuses and vasa vasorum. In one aspect of the invention, a DSCR1 modulator is introduced directly into vascular conduits supplying blood to the myocardium. Such vascular conduits include the coronary arteries as well as vessels such as saphenous veins or internal mammary artery grafts. In one embodiment, a blood vessel comprises activated endothelial cells.

"Artery" refers to a blood vessel through which blood passes away from the heart. Coronary arteries supply the tissues of the heart itself (particularly the myocardium), while other arteries supply the remaining organs of the body. The general structure of an artery consists of a lumen surrounded by a multi-layered arterial wall.

The term "angiogenesis", as used herein, refers to a cellular event resulting in neovascularization, in which vascular endothelial cells proliferate, prune and reorganize to form new vessels from preexisting vascular networks. Angiogenesis is a cascade of processes that include (1) degradation of the extracellular matrix of a local venue after the release of a protease; (2) proliferation of capillary endothelial cells, and (3) migration of capillary tubules toward the angiogenic stimulus. See, e.g., Ferrara et al., Endocrine Rev. (1992), 13:18-32; Ferrara, Nature Reviews (2002), 2:795-803.

As used herein, the phrase "vascular integrity", and variations thereof, refers to an unimpaired state of the vasculature, wherein an unimpaired state includes having a normal physiological ability of the vasculature to act as a barrier for free movement of molecules between the two compartments/sides of the barrier. More specifically, the two compartments/sides generally relate to the lumen of the vasculature and the extralumenal space (which generally comprises tissues and/or cells adjacent the the vasculature).

As used herein, the phrase "perturbations of vascular integrity", and variations thereof, refers to an abnormal and/or undesirable change to vascular integrity that results in inability of the vasculature to act as a barrier for movement between the two compartments of the barrier. For example, inflammation of the vasculature is a common cause associated with perturbations of vascular integrity. Perturbations of vascular integrity can be manifested in the form of one or more tissue and cellular conditions, including but not limited to those associated with endothelial cell necrosis, endothelial cell apoptosis, trauma to the endothelium, injury, vascular leakage, hypertension, and vascular damage. A further example include trauma affecting the vascular endothelium, e.g. trauma (such as injuries) to the blood vessels, including the vascular network of organs, to which an animal (generally a mammal) or human is subjected; such trauma would include wounds, incisions, and ulcers (e.g. diabetic ulcers) and wounds or lacerations of the blood vessels/endothelial cells. Trauma includes conditions caused by internal events as well as those that are imposed by an extrinsic agent such as a pathogen.

As used herein, "disorder associated with perturbation of vascular integrity", "disorder associated with abnormal vascular inflammation", and variations thereof, generally refer to pathological conditions thought or known to be associated with perturbation of vascular integrity and/or abnormal vascular inflammation. These disorders include, but are not limited to, vascular (such as cardiovascular) disorder, endothelial cell disorder, angiogenic disorder (e.g., cancer), and immunological disorder (e.g., inflammatory disorder, including automimmune disease). For example, these disorders include pathological conditions associated with dysregulation of angiogenesis. Disorders that can be treated by modulator molecules and methods of the invention include, but are not limited to, undesired or aberrant hypertrophy, arthritis, rheumatoid arthritis (RA), psoriasis, psoriatic plaques, sarcoidosis, atherosclerosis, atherosclerotic plaques, edema from myocardial infarction, diabetic and other proliferative retinopathies including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, chronic inflammation, lung inflammation, acute lung injury/ARDS, sepsis, hypertension (e.g., primary pulmonary hypertension), malignant pulmonary effusions, cerebral edema (e.g., associated with acute stroke/ closed head injury/ trauma), synovial inflammation, pannus formation in RA, myositis ossificans, hypertropic bone formation, osteoarthritis (OA), refractory ascites, polycystic ovarian disease, endometriosis, 3rd spacing of fluid diseases (pancreatitis, compartment syndrome, bums, bowel disease), uterine fibroids, premature labor, chronic inflammation such as IBD (Crohn's disease and ulcerative colitis), renal allograft rejection, inflammatory bowel disease, nephrotic syndrome, undesired or aberrant tissue mass growth (non-cancer), obesity, adipose tissue mass growth, hemophilic joints, hypertrophic scars, inhibition of hair growth, Osler-Weber syndrome, pyogenic granuloma retrolental fibroplasias, scleroderma, trachoma, vascular adhesions, synovitis, dermatitis, preeclampsia, ascites, pericardial effusion (such as that associated with pericarditis), pleural effusion, gastrointestinal ulceration, chronic airway inflammation and vasculitis.

Dysregulation of angiogenesis can lead to many disorders that can be treated by compositions and methods of the invention. These disorders include both non-neoplastic and neoplastic conditions. Neoplastic disorders include but are not limited to those described above. Non-neoplastic disorders include but are not limited to undesired or aberrant hypertrophy, arthritis, rheumatoid arthritis (RA), psoriasis, psoriatic plaques, sarcoidosis, atherosclerosis, atherosclerotic plaques, diabetic and other proliferative retinopathies including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, chronic inflammation, lung inflammation, acute lung injury/ARDS, sepsis, primary pulmonary hypertension, malignant pulmonary effusions, cerebral edema (e.g., associated with acute stroke/ closed head injury/ trauma), synovial inflammation, pannus formation in RA, myositis ossificans, hypertropic bone formation, osteoarthritis (OA), refractory ascites, polycystic ovarian disease, endometriosis, 3rd spacing of fluid diseases (pancreatitis, compartment syndrome, bums, bowel disease), uterine fibroids, premature labor, chronic inflammation such as IBD (Crohn's disease and ulcerative colitis), renal allograft rejection, inflammatory bowel disease, nephrotic syndrome, undesired or aberrant tissue mass growth (non-cancer), hemophilic joints, hypertrophic scars, inhibition of hair growth, Osler-Weber syndrome, pyogenic granuloma retrolental fibroplasias, scleroderma, trachoma, vascular adhesions, synovitis, dermatitis, preeclampsia, ascites, pericardial effusion (such as that associated with pericarditis), and pleural effusion.

A "disorder" is any condition that would benefit from treatment with a DSCR1 modulator and/or method of the invention. This includes chronic and acute disorders or diseases including those pathological conditions which predispose the mammal to the disorder in question. Non-limiting examples of disorders to be treated herein include malignant and benign tumors; non-leukemias and lymphoid malignancies; neuronal, glial, astrocytal, hypothalamic and other glandular, macrophagal, epithelial, stromal and blastocoelic disorders; and inflammatory (e.g., autoimmune), angiogenic and immunologic disorders.

An "autoimmune disease" herein is a non-malignant disease or disorder arising from and directed against an individual's own tissues. The autoimmune diseases herein specifically exclude malignant or cancerous diseases or conditions, especially excluding B cell lymphoma, acute lymphoblastic leukemia (ALL), chronic lymphocytic leukemia (CLL), Hairy cell leukemia and chronic myeloblastic leukemia. Examples of autoimmune diseases or disorders include, but are not limited to, inflammatory responses such as inflammatory skin diseases including psoriasis and dermatitis (*e*.*g*. atopic dermatitis); systemic scleroderma and sclerosis; responses associated with inflammatory bowel disease (such as Crohn's disease and ulcerative colitis); respiratory distress syndrome (including adult respiratory distress syndrome; ARDS); dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE); diabetes mellitus (*e*.*g*. Type I diabetes mellitus or insulin dependent diabetes mellitis); multiple sclerosis; Reynaud's syndrome; autoimmune thyroiditis; allergic encephalomyelitis; Sjorgen's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia (including, but not limited to cryoglobinemia or Coombs positive anemia) ; myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia etc.

An "angiogenic factor" or "angiogenic agent", as used herein, is a molecule which stimulates the development of blood vessels, e.g., promotes angiogenesis, endothelial cell growth, stability of blood vessels, and/or vasculogenesis, etc. In one embodiment, an angiogenic factor referred to in a claimed invention is VEGF and members of the VEGF family (A, B, C, D, and E). In one embodiment, an angiogenic factor referred to in a claimed invention is a member of the PIGF and/or PDGF family. In one embodiment, an angiogenic factor referred to in a claimed invention is a TIE ligand (Angiopoietins). In one embodiment, an angiogenic factor referred to in a claimed invention is ephrin. In one embodiment, an angiogenic factor referred to in a claimed invention is a factor that accelerates wound healing, including but not limited to one or more of growth hormone, insulin-like growth factor-I (IGF-1), VIGF, epidermal growth factor (EGF), CTGF and members of its family, and TGF-α and TGF-β. *See, e*.*g*., Klagsbrun and D'Amore, Annu. Rev. Physiol., 53:217-39 (1991); Streit and Detmar, Oncogene, 22:3172-3179 (2003); Ferrara & Alitalo, Nature Medicine 5(12):1359-1364 (1999); Tonini et al., Oncogene, 22:6549-6556 (2003) (e.g., Table 1 listing known angiogenic factors); and Sato Int. J. Clin. Oncol., 8:200-206 (2003).

The phrase "side effects", or variations thereof, as used herein refers to clinical, medical, physical, physiological and/or biochemical effects that are measurable and/or observable in a subject undergoing treatment of a disorder, wherein the effects are not part of the intended treatment outcome. Generally, the effects are undesirable with respect to the state of health and/or comfort of the treated subject, health risks for the treated subject, and/or tolerability of the treatment for the treated subject.

As described above, in treating inflammatory diseases (e.g. autoimmune diseases or autoimmune related conditions) described herein, a subject can be treated with a DSCR1 modulator of the invention, in conjunction with a second therapeutic agent, such as an immunosuppressive agent (i.e., an anti-inflammatory agent), such as in a multi drug regimen. The DSCR1 modulator can be administered concurrently, sequentially or alternating with the immunosuppressive agent. The immunosuppressive agent can be administered at the same or lesser dosages than as set forth in the art. The preferred adjunct immunosuppressive agent will depend on many factors, including the type of disorder being treated as well as the patient's history.

"Immunosuppressive agent", as used herein, refers to substances that act to suppress or mask the immune system and/or inflammatory response of a patient. Such agents would include substances that suppress cytokine production, down regulate or suppress self-antigen expression, or mask the MHC antigens. Examples of such agents include steroids such as glucocorticosteroids, *e*.*g*., prednisone, methylprednisolone, and dexamethasone; 2-amino-6-aryl-5-substituted pyrimidines (see U.S. Pat. No. 4,665,077), azathioprine (or cyclophosphamide, if there is an adverse reaction to azathioprine); bromocryptine; glutaraldehyde (which masks the MHC antigens, as described in U.S. Pat. No. 4,120,649); anti-idiotypic antibodies for MHC antigens and MHC fragments; cyclosporin A; cytokine or cytokine receptor antagonists including anti-interferon-γ, -β, or -α antibodies; anti-tumor necrosis factor-α antibodies; anti-tumor necrosis factor-β antibodies; anti-interleukin-2 antibodies and anti-IL-2 receptor antibodies; anti-L3T4 antibodies; heterologous antilymphocyte globulin; pan-T antibodies, preferably anti-CD3 or anti-CD4/CD4a antibodies; soluble peptide containing a LFA-3 binding domain (WO 90/08187 published 7/26/90); streptokinase; TGF-β; streptodornase; RNA or DNA from the host; FK506; RS-61443; deoxyspergualin; rapamycin; T-cell receptor (U.S. Pat. No. 5,114,721); T-cell receptor fragments (Offner et al., Science 251:430-432 (1991); WO 90/11294; and WO 91/01133); and T cell receptor antibodies (EP 340,109) such as T10B9.

The term "DSCR1" as used herein encompasses native sequence polypeptides, polypeptide variants and fragments of a native sequence polypeptide and polypeptide variants (which are further defined herein) that is capable of modulating calcineurin (and, for example, expression of inflammatory marker genes such as tissue factor, E-selectin, and Cox-2) in a manner similar to wild type DSCR1. The DSCR1 polypeptide described herein may be that which is isolated from a variety of sources, such as from human tissue types or from another source, or prepared by recombinant or synthetic methods. The terms "DSCR1", "DSCR1, polypeptide", "DSCR1 protein", and "DSCR1 molecule" also include variants of a DSCR1 polypeptide as disclosed herein. A "DSCR1 modulator" of the invention is a molecule that modulates the normal biological function/activity of a "DSCR1 polypeptide" or "DSCR1 protein."

A "native sequence DSCR1 polypeptide" comprises a polypeptide having the same amino acid sequence as the corresponding DSCR1 polypeptide derived from nature. In one embodiment, a native sequence DSCR1 polypeptide comprises the protein coding amino acid sequence of SEQ ID NO:1 (see Figure 9), wherein the first methionine is amino acid position 1. Such native sequence DSCR1 polypeptide can be isolated from nature or can be produced by recombinant or synthetic means. The terms "DSCR1 polypeptide" and "DSCR1 protein", as used herein, specifically encompass naturally-occurring truncated or otherwise post-translationally modified forms of the specific DSCR1 polypeptide, naturally-occurring variant forms (e.g., alternatively spliced forms) and naturally-occurring allelic variants of the polypeptide.

"DSCR1 polypeptide variant", or variations thereof, means a DSCR1 polypeptide, generally an active DSCR1 polypeptide, as defined herein having at least about 80% amino acid sequence identity with a native sequence DSCR1 polypeptide sequence as disclosed herein. Such DSCR1 polypeptide variants include, for instance, DSCR1 polypeptides wherein one or more amino acid residues are added, or deleted, at the N- or C-terminus of a native amino acid sequence. Ordinarily, a DSCR1 polypeptide variant will have at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% amino acid sequence identity, to a native sequence DSCR1 polypeptide sequence as disclosed herein. Ordinarily, DSCR1 variant polypeptides are at least about 10 amino acids in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 70, 480, 490, 500, 510, 520, 530, 540, 550, 560, 570, 580, 590, 600 amino acids in length, or more. Optionally, DSCR1 variant polypeptides will have no more than one conservative amino acid substitution as compared to a native DSCR1 polypeptide sequence, alternatively no more than 2, 3, 4, 5, 6, 7, 8, 9, or 10 conservative amino acid substitution as compared to the native DSCR1 polypeptide sequence.

"Percent (%) amino acid sequence identity" with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, as described in US Pat. No. 6,828,146.

As used herein, the terms "peptide" and "polypeptide" are used interchangeably, except that the term "peptide" generally refers to polypeptide comprising fewer than 200 contiguous amino acids.

The term "vector," as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "recombinant vectors"). In general, expression vectors of utility in recombinant DNA techniques are often in the form of plasmids. In the present specification, "plasmid" and "vector" may be used interchangeably as the plasmid is the most commonly used form of vector.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, .alpha.-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR.sub.2 ("amidate"), P(O)R, P(O)OR', CO or CH.sub.2 ("formacetal"), in which each R or R' is independently H or substituted or unsubstituted alkyl (1-20 C.) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

The term "host cell" (or "recombinant host cell"), as used herein, is intended to refer to a cell that has been genetically altered, or is capable of being genetically altered by introduction of an exogenous polynucleotide, such as a recombinant plasmid or vector. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which generally lack antigen specificity. Polypeptides of the latter kind are, for example, produced at low levels by the lymph system and at increased levels by myelomas.

The terms "antibody" and "immunoglobulin" are used interchangeably in the broadest sense and include monoclonal antibodies (*e*.*g*., full length or intact monoclonal antibodies), polyclonal antibodies, monovalent, multivalent antibodies, multispecific antibodies (*e*.*g*., bispecific antibodies so long as they exhibit the desired biological activity) and may also include certain antibody fragments (as described in greater detail herein). An antibody can be chimeric, human, humanized and/or affinity matured.

"Antibody fragments" comprise only a portion of an intact antibody, wherein the portion preferably retains at least one, preferably most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an *in vivo* half life substantially similar to an intact antibody. For example, such an antibody fragment may comprise on antigen binding arm linked to an Fc sequence capable of conferring *in vivo* stability to the fragment.

The "variable region" or "variable domain" of an antibody refers to the amino-terminal domains of heavy or light chain of the antibody. These domains are generally the most variable parts of an antibody and contain the antigen-binding sites.

The term "hypervariable region", "HVR", or "HV", when used herein refers to the regions of an antibody variable domain which are hypervariable in sequence and/or form structurally defined loops. Generally, antibodies comprise six hypervariable regions; three in the VH (H1, H2, H3), and three in the VL (L1, L2, L3). A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk J. Mol. Biol. 196:901-917 (1987)). The AbM hypervariable regions represent a compromise between the Kabat CDRs and Chothia structural loops, and are used by Oxford Molecular's AbM antibody modeling software. The "contact" hypervariable regions are based on an analysis of the available complex crystal structures. The residues from each of these hypervariable regions are noted below.

| Loop Kabat | | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B |

| (Kabat Numbering) | | | | |
|---|---|---|---|---|
| H1 | H31-H35 | H26-H35 | H26-H32 | H30-H35 |

| (Chothia Numbering) | | | | |
|---|---|---|---|---|
| H2 | H50-H65 | H50-H58 | H53-H55 | H47-H58 |
| H3 | H95-H102 | H95-H102 | H96-H101 | H93-H101 |

"Framework" or "FR" residues are those variable domain residues other than the hypervariable region residues as herein defined.

The term "monoclonal antibody" as used herein refers to an antibody obtained from a population of substantially homogeneous antibodies, *i*.*e*., the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (*e*.*g*., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

An "affinity matured" antibody is one with one or more alterations in one or more CDRs/HVRs thereof which result in an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR/HVR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

The term "Fc region" is used to define the C-terminal region of an immunoglobulin heavy chain which may be generated by papain digestion of an intact antibody. The Fc region may be a native sequence Fc region or a variant Fc region. Although the boundaries of the Fc region of an immunoglobulin heavy chain might vary, the human IgG heavy chain Fc region is usually defined to stretch from an amino acid residue at about position Cys226, or from about position Pro230, to the carboxyl-terminus of the Fc region. The Fc region of an immunoglobulin generally comprises two constant domains, a CH2 domain and a CH3 domain, and optionally comprises a CH4 domain. By "Fc region chain" herein is meant one of the two polypeptide chains of an Fc region.

The phrase "substantially similar" or "substantially equivalent", as used herein, denotes a sufficiently high degree of similarity between two numeric values such that one of skill in the art would consider the difference between the two values to be of little or no biological significance within the context of the biological characteristic measured by said values. The difference between said two values is preferably less than about 50%, preferably less than about 40%, preferably less than about 30%, preferably less than about 20%, preferably less than about 10%.

The term "cytotoxic agent" as used herein refers to a substance that inhibits or prevents the function of cells and/or causes destruction of cells. The term is intended to include radioactive isotopes (e.g. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®) and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., paclitaxel (TAXOL®), albumin-engineered nanoparticle formulation of paclitaxel (ABRAXANE^{™}), and doxetaxel (TAXOTERE®); chloranbucil; 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluorometlhylornithine (DMFO); retinoids such as retinoic acid; pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN^{™}) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX® tamoxifen), raloxifene (EVISTA®), droloxifene, 4-hydroxytamoxifen, trioxifene, keoxifene, LY117018, onapristone, and toremifene (FARESTON®); anti-progesterones; estrogen receptor down-regulators (ERDs); estrogen receptor antagonists such as fulvestrant (FASLODEX®); agents that function to suppress or shut down the ovaries, for example, leutinizing hormone-releasing hormone (LHRH) agonists such as leuprolide acetate (LUPRON® and ELIGARD®), goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, megestrol acetate (MEGASE®), exemestane (AROMASIN®), formestanie, fadrozole, vorozole (RIVISOR®), letrozole (FEMARA®), and anastrozole (ARIMIDEX®). In addition, such definition of chemotherapeutic agents includes bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor (e.g., LURTOTECAN®); rmRH (e.g., ABARELIX®); lapatinib ditosylate (an ErbB-2 and EGFR dual tyrosine kinase small-molecule inhibitor also known as GW572016); COX-2 inhibitors such as celecoxib (CELEBREX®; 4-(5-(4-methylphenyl)-3-(trifluoromethyl)-1H-pyrazol-1-yl) benzenesulfonamide; and pharmaceutically acceptable salts, acids or derivatives of any of the above.

A "blocking" antibody or an "antagonist" antibody is one which inhibits or reduces biological activity of the antigen it binds. Such blocking can occur by any means, *e*.*g*. by interfering with protein-protein interaction such as ligand binding to a receptor. In on embodiment, blocking antibodies or antagonist antibodies substantially or completely inhibit the biological activity of the antigen.

The terms "cancer" and "cancerous" refer to or describe the physiological condition in mammals that is typically characterized by unregulated cell growth/proliferation. Examples of cancer include but are not limited to, carcinoma, lymphoma (e.g., Hodgkin's and non-Hodgkin's lymphoma), blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma and various types of head and neck cancer.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, modulator molecules and methods of the invention are used to delay development of a disease or disorder.

An "effective amount" refers to an amount effective, at dosages and for periods of time necessary, to achieve the desired therapeutic or prophylactic result. A "therapeutically effective amount" of a therapeutic agent may vary according to factors such as the disease state, age, sex, and weight of the individual, and the ability of the antibody to elicit a desired response in the individual. A therapeutically effective amount is also one in which any toxic or detrimental effects of the therapeutic agent are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

### II. Compositions and Methods of the Invention

### A. DSCR1 Modulator Antibodies

In one embodiment, the present invention provides DSCR1 modulator antibodies which may find use herein as therapeutic and/or diagnostic agents. Exemplary antibodies include polyclonal, monoclonal, humanized, bispecific, and heteroconjugate antibodies. Aspects of generating, identifying, characterizing, modifying and producing antibodies are well established in the art, e.g., as described in US Pat. Appl. Pub. No. 2005/0042216 from paragraphs 522 through 563, 604 through 608, and 617 through 688.

The DSCR1 modulator antibodies disclosed herein can be formulated in any suitable form for delivery to a target cell/tissue. For example, the antibodies may be formulated as immunoliposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA 82:3688 (1985); Hwang et al., Proc. Natl Acad. Sci. USA 77:4030 (1980); U.S. Pat. Nos. 4,485,045 and 4,544,545; and WO97/38731 published October 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem. 257:286-288 (1982) via a disulfide interchange reaction. A chemotherapeutic agent is optionally contained within the liposome. See Gabizon et al., J. National Cancer Inst. 81(19):1484 (1989).

### B. DSCR1 Modulator Polypeptides

In one aspect, a DSCR1 modulator of the invention comprises a polypeptide. In one embodiment, the modulator polypeptide antagonizes NFAT activity in an endothelial cell. In one embodiment, the modulator polypeptide antagonizes CnA activity in an endothelial cell. In one embodiment, the polypeptides bind, preferably specifically, to DSCR1 or an intracellular molecule that interacts with DSCR1 or CnA. The polypeptides may be chemically synthesized using known peptide synthesis methodology or may be prepared and purified using recombinant technology. In one embodiment, a DSCR1 modulator polypeptide is at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 1, 52, 53, 54, 5, 56, 7, 58, 9, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such polypeptides are capable of modulating DSCR1 activity. These polypeptides may be identified without undue experimentation using well known techniques. In this regard, it is noted that techniques for screening oligopeptide libraries for oligopeptides that are capable of specifically binding to a polypeptide target are well known in the art (see, e.g., U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci. U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. (1990) Proc. Natl. Acad. Sci. USA, 87:6378; Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668).

In this regard, bacteriophage (phage) display is one well known technique which allows one to screen large oligopeptide libraries to identify member(s) of those libraries which are capable of specifically binding to a polypeptide target. Phage display is a technique by which variant polypeptides are displayed as fusion proteins to the coat protein on the surface of bacteriophage particles (Scott, J.K. and Smith, G. P. (1990) Science, 249: 386). The utility of phage display lies in the fact that large libraries of selectively randomized protein variants (or randomly cloned cDNAs) can be rapidly and efficiently sorted for those sequences that bind to a target molecule with high affinity. Display of peptide (Cwirla, S. E. et al. (1990) Proc. Natl. Acad. Sci. USA, 87:6378) or protein (Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363) libraries on phage have been used for screening millions of polypeptides or oligopeptides for ones with specific binding properties (Smith, G. P. (1991) Current Opin. Biotechnol., 2:668). Sorting phage libraries of random mutants requires a strategy for constructing and propagating a large number of variants, a procedure for affinity purification using the target receptor, and a means of evaluating the results of binding enrichments. U.S. Patent Nos. 5,223,409, 5,403,484, 5,571,689, and 5,663,143.

Although most phage display methods have used filamentous phage, lambdoid phage display systems (WO 95/34683; U.S. 5,627,024), T4 phage display systems (Ren et al., Gene, 215: 439 (1998); Zhu et al., Cancer Research, 58(15): 3209-3214 (1998); Jiang et al., Infection & Immunity, 65(11): 4770-4777 (1997); Ren et al., Gene, 195(2):303-311 (1997); Ren, Protein Sci., 5: 1833 (1996); Efimov et al., Virus Genes, 10: 173 (1995)) and T7 phage display systems (Smith and Scott, Methods in Enzymology, 217: 228-257 (1993); U.S. 5,766,905) are also known.

Many other improvements and variations of the basic phage display concept have now been developed. These improvements enhance the ability of display systems to screen peptide libraries for binding to selected target molecules and to display functional proteins with the potential of screening these proteins for desired properties.

Combinatorial reaction devices for phage display reactions have been developed (WO 98/14277) and phage display libraries have been used to analyze and control bimolecular interactions (WO 98/20169; WO 98/20159) and properties of constrained helical peptides (WO 98/20036). WO 97/35196 describes a method of isolating an affinity ligand in which a phage display library is contacted with one solution in which the ligand will bind to a target molecule and a second solution in which the affinity ligand will not bind to the target molecule, to selectively isolate binding ligands. WO 97/46251 describes a method of biopanning a random phage display library with an affinity purified antibody and then isolating binding phage, followed by a micropanning process using microplate wells to isolate high affinity binding phage. The use of *Staphlylococcus aureus* protein A as an affinity tag has also been reported (Li et al. (1998) Mol Biotech., 9:187). WO 97/47314 describes the use of substrate subtraction libraries to distinguish enzyme specificities using a combinatorial library which may be a phage display library. A method for selecting enzymes suitable for use in detergents using phage display is described in WO 97/09446. Additional methods of selecting specific binding proteins are described in U.S. Patent Nos. 5,498,538, 5,432,018, and WO 98/15833.

Methods of generating peptide libraries and screening these libraries are also disclosed in U.S. Patent Nos. 5,723,286, 5,432,018, 5,580,717, 5,427,908, 5,498,530, 5,770,434, 5,734,018, 5,698,426, 5,763,192, and 5,723,323.

Methods of generating, identifying, characterizing, modifying and producing modulator polypeptides are well established in the art, e.g., as described in US Pat. Appl. Pub. No. 2005/0042216 from paragraphs 606 through 608, 614 through 688.

In one embodiment, polypeptides for antagonizing DSCR1-dependent CnA Activity can be designed based on DSCR1 domain structure, e.g. as described in Rothermel et al., Trends Cardiovascular Med. (2003), 13(1):15-21; Vega et al., J. Biol. Chem. (2002), 277(33):30401-30407. For example, the polypeptide may comprise amino acid from about position 96 to about 125, or about 108 to about 122 of human DSCR1. In another embodiment, the polypeptide comprises at least a portion of the serine-proline (SP) sequence and at least a portion of the N and/or C-terminal calcineurin binding domain of human DSCR1, wherein the polypeptide is capable of binding to CnA to inhibit CnA-regulated gene expression.

### C. DSCR1 Modulator Small Molecules

In one embodiment, DSCR1 modulator small molecules are organic molecules other than oligopeptides or antibodies as defined herein that modulate DSCR1 activity. DSCR1 modulator small molecules may be identified and chemically synthesized using known methodology (see, e.g., PCT Publication Nos. WO00/00823 and WO00/39585). DSCR1, modulator organic small molecules are usually less than about 2000 daltons in size, alternatively less than about 1500, 750, 500, 250 or 200 daltons in size, wherein such organic small molecules may be identified without undue experimentation using well known techniques. In this regard, it is noted that techniques for screening organic small molecule libraries for molecules that are capable of binding to a polypeptide target are well known in the art (see, e.g., PCT Publication Nos. WO00/00823 and WO00/39585). DSCR1 modulator organic small molecules may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, acid chlorides, or the like.

### D. DSCR1 Modulators Comprising Nucleic Acids

In one aspect, a DSCR1 modulator of the invention comprises a nucleic acid molecule. For example, the nucleic acid molecule may comprise a sense/antisense oligonucleotide, an inhibitory/interfering RNA (e.g., a small inhibitory/interfering RNA (siRNA)), or an aptamer. Methods for screening for, identifying and making these nucleic acid modulators are known in the art.

For example, siRNAs have proven capable of modulating gene expression where traditional antagonists such as small molecules or antibodies have failed. (Shi Y., Trends in Genetics 19(1):9-12 (2003)). In vitro synthesized, double stranded RNAs that are fewer than 30 nucleotides in length (e.g., about 15 to 25, 17 to 20, 18 to 20, 19 to 20, or 21 to 23 nucleotides) can act as interfering RNAs (iRNAs) and can specifically inhibit gene expression (see, e.g., Fire A., Trends in Genetics (1999), 391; 806-810; US Pat. Applns. 09/821832, 09/215257; US Pat. No. 6,506,559; PCT/US01/10188; European Appln. Ser. No. 00126325). These iRNAs are believed to act at least in part by mediating degradation of their target RNAs. However, since they are under 30 nuclotides in length, they do not trigger a cell antiviral defense mechanism. Such mechanisms include interferon production, and a general shutdown of host cell protein synthesis. Practically, siRNAs can be synthesized and then cloned into DNA vectors. Such vectors can be transfected and made to express the siRNA at high levels. The high level of siRNA expression is used to "knockdown" or significantly reduce the amount of protein produced in a cell, and thus it is useful in cellular settings where overexpression of a protein is believed to be linked to a pathological disorder.

Aptamers are nucleic acid molecules that are capable of binding to a target molecule, such as a DSCR1 protein. The generation and therapeutic use of aptamers are well established in the art. See, e.g., US Pat. No. 5,475,096, and the therapeutic efficacy of Macugen® (Eyetech, New York) for treating age-related macular degeneration.

Anti-sense technology is well established in the art. Further details regarding this technology are provided hereinbelow.

### E. Pharmaceutical Formulations

Therapeutic formulations of the DSCR1 modulators used in accordance with the invention are prepared for storage by mixing the DSCR1 modulator having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as acetate, Tris, phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; tonicifiers such as trehalose and sodium chloride; sugars such as sucrose, mannitol, trehalose or sorbitol; surfactant such as polysorbate; salt-forming counter-ions such as sodium; metal complexes (e.g., Zn-protein complexes); and/or non-ionic surfactants such as TWEEN®, PLURONICS® or polyethylene glycol (PEG).

The formulations herein may also contain more than one active compound as necessary for the particular indication being treated, preferably those with complementary activities that do not adversely affect each other. For example, in addition to a DSCR1 modulator, it may be desirable to include in the one formulation, an additional modulator, e.g., a second antibody which binds a different epitope on the DSCR1 protein, or an antibody to some other target. Alternatively, or additionally, the composition may further comprise a chemotherapeutic agent, cytotoxic agent, cytokine, growth inhibitory agent, anti-hormonal agent, and/or cardioprotectant. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. Ed. (1980).

Sustained-release preparations may be prepared. Suitable examples of sustained-release preparations include semi-permeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT® (injectable microspheres composed of lactic acid-glycolic acid copolymer and leuprolide acetate), and poly-D-(-)-3-hydroxybutyric acid.

The formulations to be used for *in vivo* administration must be sterile. This is readily accomplished by filtration through sterile filtration membranes.

### F. Treatment with a DSCR1 modulator of the invention

DSCR1 modulators of the invention have various non-therapeutic applications. The modulators can be useful for staging or detecting DSCR1-expressing diseases (e.g., in radioimaging). The antibodies, oligopeptides and organic small molecules are also useful for purification or immunoprecipitation of DSCR1 from cells, for detection and quantitation of DSCR1 *in vitro,* e.g., in an ELISA or a Western blot, to modulate cellular events in a population of cells.

Currently, depending on the stage of the cancer, cancer treatment involves one or a combination of the following therapies: surgery to remove the cancerous tissue, radiation therapy, and chemotherapy. Therapy comprising DSCR1 modulators may be especially desirable in elderly patients who do not tolerate the toxicity and side effects of chemotherapy well and in metastatic disease where radiation therapy has limited usefulness. For therapeutic applications, the DSCR1 modulators can be used alone, or in combination therapy with, e.g., hormones, antiangiogens, or radiolabelled compounds, or with surgery, cryotherapy, and/or radiotherapy. The DSCR1 modulators can be administered in conjunction with other forms of conventional therapy, either consecutively with, pre- or post-conventional therapy. Chemotherapeutic drugs such as TAXOTERE® (docetaxel), TAXOL® (palictaxel), estramustine and mitoxantrone are used in treating cancer, in particular, in good risk patients. The DSCR1 modulators would generally be administered with a therapeutically effective dose of the chemotherapeutic agent. In another embodiment, a DSCR1 modulator is administered in conjunction with chemotherapy to reduce side-effects reslting from the chemotherapeutic agent, e.g., paclitaxel. The Physicians' Desk Reference (PDR) discloses dosages of these agents that have been used in treatment of various cancers. The dosing regimen and dosages of these aforementioned chemotherapeutic drugs that are therapeutically effective will depend on the particular cancer being treated, the extent of the disease and other factors familiar to the physician of skill in the art and can be determined by the physician.

The DSCR1 modulators are administered to a human patient, in accordance with known methods, such as intravenous administration, e.g.,, as a bolus or by continuous infusion over a period of time, by intramuscular, intraperitoneal, intracerobrospinal, subcutaneous, intra-articular, intrasynovial, intrathecal, oral, topical, or inhalation routes. Intravenous or subcutaneous administration of the antibody, oligopeptide or organic small molecule is preferred in one embodiment of the invention.

Other therapeutic regimens may be combined with the administration of the DSCR1 modulator. The combined administration includes co-administration, using separate formulations or a single pharmaceutical formulation, and consecutive administration in either order, wherein preferably there is a time period while both (or all) active agents simultaneously exert their biological activities. Preferably such combined therapy results in a synergistic therapeutic effect and/or reduction of unwanted side effects.

It may also be desirable to combine administration of the DSCR1 modulator, with administration of a therapeutic agent directed against another antigen associated with the particular pathological condition.

In another embodiment, the therapeutic treatment methods of the present invention involves the combined administration of a DSCR1 modulator molecule and one or more chemotherapeutic agents or growth inhibitory agents, including co-administration of cocktails of different chemotherapeutic agents. Chemotherapeutic agents include estramustine phosphate, prednimustine, cisplatin, 5-fluorouracil, melphalan, cyclophosphamide, hydroxyurea and hydroxyureataxanes (such as paclitaxel and doxetaxel) and/or anthracycline antibiotics. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992).

The DSCR1 modulator may be combined with an anti-hormonal compound; e.g., an anti-estrogen compound such as tamoxifen; an anti-progesterone such as onapristone (see, EP 616 812); or an anti-androgen such as flutamide, in dosages known for such molecules. Where the cancer to be treated is androgen independent cancer, the patient may previously have been subjected to anti-androgen therapy and, after the cancer becomes androgen independent, the DSCR1 modulator may be administered to the patient.

Sometimes, it may be beneficial to also co-administer a cardioprotectant (to prevent or reduce myocardial dysfunction associated with the therapy) or one or more cytokines to the patient. In addition to the above therapeutic regimes, the patient may be subjected to surgical removal of cancer cells and/or radiation therapy, before, simultaneously with, or post DSCR1 modulator therapy. Suitable dosages for any of the above co-administered agents are those presently used and may be lowered due to the combined action (synergy) of the agent and DSCR1 modulator.

For the prevention or treatment of disease, the dosage and mode of administration will be chosen by the physician according to known criteria. The appropriate dosage of DSCR1 modulator will depend on the type of disease to be treated, as defined above, the severity and course of the disease, whether the DSCR1 modulator is administered for preventive or therapeutic purposes, previous therapy, the patient's clinical history and response to the DSCR1 modulator, and the discretion of the attending physician. The DSCR1 modulator is suitably administered to the patient at one time or over a series of treatments. In one embodiment, the DSCR1 modulator is administered by intravenous infusion or by subcutaneous injections. Depending on the type and severity of the disease, about 1 µg/kg to about 50 mg/kg body weight (e.g., about 0.1-15mg/kg/dose) of antibody can be an initial candidate dosage for administration to the patient, whether, for example, by one or more separate administrations, or by continuous infusion. A dosing regimen can comprise administering an initial loading dose of about 4 mg/kg, followed by a weekly maintenance dose of about 2 mg/kg of the DSCR1 modulator antibody. However, other dosage regimens may be useful. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. The progress of this therapy can be readily monitored by conventional methods and assays and based on criteria known to the physician or other persons of skill in the art.

Aside from administration of a polypeptide modulator (e.g., polypeptide, antibody, etc.) to the patient, the invention contemplates administration of a modulator by gene therapy. Such administration of nucleic acid comprising/encoding the DSCR1 modulator is encompassed by the expression "administering a therapeutically effective amount of a DSCR1 modulator". See, for example, WO96/07321 published March 14, 1996 concerning the use of gene therapy to generate intracellular antibodies.

There are two major approaches to getting the nucleic acid (optionally contained in a vector) into the patient's cells; *in vivo* and *ex vivo.* For *in vivo* delivery the nucleic acid is injected directly into the patient, usually at the site where the DSCR1 modulator is required. For *ex vivo* treatment, the patient's cells are removed, the nucleic acid is introduced into these isolated cells and the modified cells are administered to the patient either directly or, for example, encapsulated within porous membranes which are implanted into the patient (see, e.g., U.S. Patent Nos. 4,892,538 and 5,283,187). There are a variety of techniques available for introducing nucleic acids into viable cells. The techniques vary depending upon whether the nucleic acid is transferred into cultured cells *in vitro*, or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. A commonly used vector for *ex vivo* delivery of the gene is a retroviral vector.

In one embodiment, *in vivo* nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). For review of the currently known gene marking and gene therapy protocols see Anderson et al., Science 256:808-813 (1992). See also WO 93/25673 and the references cited therein.

DSCR1 modulator antibodies of the invention can be in the different forms encompassed by the definition of "antibody" herein. Thus, the antibodies include full length or intact antibody, antibody fragments, native sequence antibody or amino acid variants, humanized, chimeric or fusion antibodies, and functional fragments thereof.

The invention provides a composition comprising a DSCR1 modulator, and a carrier. In a further embodiment, a composition can comprise a DSCR1 modulator in combination with other therapeutic agents such as cytotoxic or growth inhibitory agents, including chemotherapeutic agents. The invention also provides formulations comprising a DSCR1 modulator, and a carrier. In one embodiment, the formulation is a therapeutic formulation comprising a pharmaceutically acceptable carrier.

### G. Articles of Manufacture and Kits

Another embodiment of the invention is an article of manufacture containing materials useful for the treatment of a disorder using a DSCR1 modulator. The article of manufacture comprises a container and a label or package insert on or associated with the container. Suitable containers include, for example, bottles, vials, syringes, etc. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). At least one active agent in the composition is a DSCR1 modulator of the invention. The label or package insert indicates that the composition is used for treating a particular disorder. The label or package insert will further comprise instructions for administering the composition to the patient. Additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Kits are also provided that are useful for various purposes. Kits can be provided which contain DSCR1 modulators of the invention for detection and quantitation of DSCR1 *in vitro,* e.g., in an ELISA or a Western blot. As with the article of manufacture, the kit comprises a container and a label or package insert on or associated with the container. The container holds a composition comprising at least one DSCR1 modulator of the invention. Additional containers may be included that contain, e.g., diluents and buffers, control antibodies. The label or package insert may provide a description of the composition as well as instructions for the intended in vitro or detection use.

### H. DSCR1 modulators comprising polypeptides or nucleic acids - Specific forms and applications

In one embodiment, nucleic acids of the invention include antisense or sense oligonucleotides/polynucleotides comprising a singe-stranded nucleic acid sequence (either RNA or DNA) capable of binding to endogenous DSCR1 nucleic acids or that encode DSCR1 polypeptide. Antisense or sense oligonucleotides, according to the present invention, comprise at least a fragment of the coding region of DSCR1 DNA. Such a fragment generally comprises at least about 14 nucleotides, preferably from about 14 to 30 nucleotides. The ability to derive an antisense or a sense oligonucleotide, based upon a cDNA sequence encoding a given protein is described in, for example, Stein and Cohen (Cancer Res. 48:2659, 1988) and van der Krol et al. BioTechniques 6:958, 1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. Such methods are encompassed by the present invention. The antisense oligonucleotides thus may be used to block expression of a DSCR1 protein in endothelial cells. Antisense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences.

Preferred intragenic sites for antisense binding include the region incorporating the translation initiation/start codon (5'-AUG / 5'-ATG) or termination/stop codon (5'-UAA, 5'-UAG and 5-UGA / 5'-TAA, 5'-TAG and 5'-TGA) of the open reading frame (ORF) of the gene. These regions refer to a portion of the mRNA or gene that encompasses from about 25 to about 50 contiguous nucleotides in either direction (i.e., 5' or 3') from a translation initiation or termination codon. Other exemplary regions for antisense binding include: introns; exons; intron-exon junctions; the open reading frame (ORF) or "coding region," which is the region between the translation initiation codon and the translation termination codon; the 5' cap of an mRNA which comprises an N7-methylated guanosine residue joined to the 5'-most residue of the mRNA via a 5'-5' triphosphate linkage and includes 5' cap structure itself as well as the first 50 nucleotides adjacent to the cap; the 5' untranslated region (5'UTR), the portion of an mRNA in the 5' direction from the translation initiation codon, and thus including nucleotides between the 5' cap site and the translation initiation codon of an mRNA or corresponding nucleotides on the gene; and the 3' untranslated region (3'UTR), the portion of an mRNA in the 3' direction from the translation termination codon, and thus including nucleotides between the translation termination codon and 3' end of an mRNA or corresponding nucleotides on the gene.

Specific examples of antisense compounds useful for inhibiting expression of DSCR1 polypeptide include oligonucleotides containing modified backbones or non-natural internucleoside linkages. Oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides. Exemplary modified oligonucleotide backbones include, for example, phosphorothioates, chiral phosphorothioates, phosphorodithioates, phosphotriesters, aminoalkylphosphotri-esters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and borano-phosphates having normal 3'-5' linkages, 2'-5' linked analogs of these, and those having inverted polarity wherein one or more internucleotide linkages is a 3' to 3', 5' to 5' or 2' to 2' linkage. Exemplary oligonucleotides having inverted polarity comprise a single 3' to 3' linkage at the 3'-most internucleotide linkage i.e. a single inverted nucleoside residue which may be abasic (the nucleobase is missing or has a hydroxyl group in place thereof). Various salts, mixed salts and free acid forms are also included. Representative United States patents that teach the preparation of phosphorus-containing linkages include, but are not limited to, U.S. Pat. Nos.: 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; 5,194,599; 5,565,555; 5,527,899; 5,721,218; 5,672,697 and 5,625,050, each of which is herein incorporated by reference.

Examples of modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH.sub.2 component parts. Representative United States patents that teach the preparation of such oligonucleosides include, but are not limited to,. U.S. Pat. Nos.: 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; 5,792,608; 5,646,269 and 5,677,439, each of which is herein incorporated by reference.

In other examples of antisense oligonucleotides, both the sugar and the internucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S. Pat. Nos.: 5,539,082; 5,714,331; and 5,719,262, each of which is herein incorporated by reference. Further teaching of PNA compounds can be found in Nielsen et al., Science, 1991, 254, 1497-1500.

Examples of antisense oligonucleotides incorporate phosphorothioate backbones and/or heteroatom backbones, and in particular -CH₂-NH-O-CH₂-, -CH₂-N(CH₃)-O-CH₂-[known as a methylene (methylimino) or MMI backbone], -CH₂-O-N(CH₃)-CH₂-, -CH₂-N(CH₃)-N(CH₃)-CH₂- and -O-N(CH₃)-CH₂-CH₂- [wherein the native phosphodiester backbone is represented as -O-P-O-CH₂-] described in the above referenced U.S. Pat. No. 5,489,677, and the amide backbones of the above referenced U.S. Pat. No. 5,602,240. Additional examples are antisense oligonucleotides having morpholino backbone structures of the above-referenced U.S. Pat. No. 5,034,506.

Modified oligonucleotides may also contain one or more substituted sugar moieties. Exemplary oligonucleotides comprise one of the following at the 2' position: OH; F; O-alkyl, S-alkyl, or N-alkyl; O-alkenyl, S-alkeynyl, or N-alkenyl; O-alkynyl, S-alkynyl or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. Particularly preferred are O[(CH₂)ₙO)ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃)]₂, where n and m are from 1 to about 10. Other exemplary antisense oligonucleotides comprise one of the following at the 2' position: C₁ to C₁₀ lower alkyl, substituted lower alkyl, alkenyl, alkynyl, alkaryl, aralkyl, O-alkaryl or O-aralkyl, SH, SCH₃, OCN, Cl, Br, CN, CF₃, OCF₃, SOCH₃, SO₂ CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. One possible modification includes 2'-methoxyethoxy (2'-O-CH₂CH₂OCH₃, also known as 2'-O-(2-methoxyethyl) or 2'-MOE) (Martin et al., Helv. Chim. Acta, 1995, 78, 486-504) i.e., an alkoxyalkoxy group. A further preferred modification includes 2'-dimethylaminooxyethoxy, i.e., a O(CH₂)₂ON(CH₃)₂ group, also known as 2'-DMAOE, and 2'-dimethylaminoethoxyethoxy (also known in the art as 2'-O-dimethylaminoethoxyethyl or 2'-DMAEOE), i.e., 2'-O-CH₂-O-CH₂-N(CH₂).

A further modification includes Locked Nucleic Acids (LNAs) in which the 2'-hydroxyl group is linked to the 3' or 4' carbon atom of the sugar ring thereby forming a bicyclic sugar moiety. The linkage can be a methelyne (-CH₂-)ₙ group bridging the 2' oxygen atom and the 4' carbon atom wherein n is 1 or 2. LNAs and preparation thereof are described in WO 98/39352 and WO 99/14226.

Other modifications include 2'-methoxy (2'-O-CH₃), 2'-aminopropoxy (2'-OCH₂CH₂CH₂ NH₂), 2'-allyl (2'-CH₂-CH=CH₂), 2'-O-allyl (2'-O-CH₂-CH=CH₂) and 2'-fluoro (2'-F). The 2'-modification may be in the arabino (up) position or ribo (down) position. One 2'-arabino modification is 2'-F. Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar. Representative United States patents that teach the preparation of such modified sugar structures include, but are not limited to, U.S. Pat. Nos.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; and 5,700,920, each of which is herein incorporated by reference in its entirety.

Oligonucleotides may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃ or -CH₂-C≡CH) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine(1H-pyrimido[5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in U.S. Pat. No. 3,687,808, those disclosed in The Concise Encyclopedia Of Polymer Science And Engineering, pages 858-859, Kroschwitz, J. I., ed. John Wiley & Sons, 1990, and those disclosed by Englisch et al., Angewandte Chemie, International Edition, 1991, 30, 613. Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2.degree. C. (Sanghvi et al, Antisense Research and Applications, CRC Press, Boca Raton, 1993, pp. 276-278) and are exemplary base substitutions, e.g., when combined with 2'-O-methoxyethyl sugar modifications. Representative United States patents that teach the preparation of modified nucleobases include, but are not limited to: U.S. Pat. No. 3,687,808, as well as U.S. Pat. Nos.: 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,645,985; 5,830,653; 5,763,588; 6,005,096; 5,681,941 and 5,750,692, each of which is herein incorporated by reference.

Another modification of antisense oligonucleotides comprises chemically linking to the oligonucleotide one or more moieties or conjugates which enhance the activity, cellular distribution or cellular uptake of the oligonucleotide. The compounds of the invention can include conjugate groups covalently bound to functional groups such as primary or secondary hydroxyl groups. Conjugate groups of the invention include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugates groups include cholesterols, lipids, cation lipids, phospholipids, cationic phospholipids, biotin, phenazine, folate, phenanthridine, anthraquinone, acridine, fluoresceins, rhodamines, coumarins, and dyes. Groups that enhance the pharmacodynamic properties, in the context of this invention, include groups that improve oligomer uptake, enhance oligomer resistance to degradation, and/or strengthen sequence-specific hybridization with RNA. Groups that enhance the pharmacokinetic properties, in the context of this invention, include groups that improve oligomer uptake, distribution, metabolism or excretion. Conjugate moieties include but are not limited to lipid moieties such as a cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., dodecandiol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety. Oligonucleotides of the invention may also be conjugated to active drug substances, for example, aspirin, warfarin, phenylbutazone, ibuprofen, suprofen, fenbufen, ketoprofen, (S)-(+)-pranoprofen, carprofen, dansylsarcosine, 2,3,5-triiodobenzoic acid, flufenamic acid, folinic acid, a benzothiadiazide, chlorothiazide, a diazepine, indomethicin, a barbiturate, a cephalosporin, a sulfa drug, an antidiabetic, an antibacterial or an antibiotic. Oligonucleotide-drug conjugates and their preparation are described in U.S. patent application Ser. No. 09/334,130 (filed Jun. 15, 1999) and United States patents Nos.: 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941, each of which is herein incorporated by reference.

It is not necessary for all positions in a given compound to be uniformly modified, and in fact more than one of the aforementioned modifications may be incorporated in a single compound or even at a single nucleoside within an oligonucleotide. The present invention also includes antisense compounds which are chimeric compounds. "Chimeric" antisense compounds or "chimeras," in the context of this invention, are antisense compounds, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Chimeric antisense compounds of the invention may be formed as composite structures of two or more oligonucleotides, modified oligonucleotides, oligonucleosides and/or oligonucleotide mimetics as described above. Exemplary chimeric antisense oligonucleotides incorporate at least one 2' modified sugar (preferably 2'-O-(CH₂)₂-O-CH₃) at the 3' terminal to confer nuclease resistance and a region with at least 4 contiguous 2'-H sugars to confer RNase H activity. Such compounds have also been referred to in the art as hybrids or gapmers. Exemplary gapmers have a region of 2' modified sugars (preferably 2'-O-(CH₂)₂-O-CH₃) at the 3'-terminal and at the 5' terminal separated by at least one region having at least 4 contiguous 2'-H sugars and may incorporate phosphorothioate backbone linkages. Representative United States patents that teach the preparation of such hybrid structures include, but are not limited to, U.S. Pat. Nos. 5,013,830; 5,149,797; 5,220,007; 5,256,775; 5,366,878; 5,403,711; 5,491,133; 5,565,350; 5,623,065; 5,652,355; 5,652,356; and 5,700,922, each of which is herein incorporated by reference in its entirety.

The antisense compounds used in accordance with this invention may be conveniently and routinely made through the well-known technique of solid phase synthesis. Equipment for such synthesis is sold by several vendors including, for example, Applied Biosystems (Foster City, Calif.). Any other means for such synthesis known in the art may additionally or alternatively be employed. It is well known to use similar techniques to prepare oligonucleotides such as the phosphorothioates and alkylated derivatives. The compounds of the invention may also be admixed, encapsulated, conjugated or otherwise associated with other molecules, molecule structures or mixtures of compounds, as for example, liposomes, receptor targeted molecules, oral, rectal, topical or other formulations, for assisting in uptake, distribution and/or absorption. Representative United States patents that teach the preparation of such uptake, distribution and/or absorption assisting formulations include, but are not limited to, U.S. Pat. Nos. 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756, each of which is herein incorporated by reference.

Other examples of sense or antisense oligonucleotides include those oligonucleotides which are covalently linked to organic moieties, such as those described in WO 90/10048, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, CaPO₄-mediated DNA transfection, electroporation, or by using gene transfer vectors such as Epstein-Barr virus. In an exemplary procedure, an antisense or sense oligonucleotide is inserted into a suitable retroviral vector. A cell containing the target nucleic acid sequence is contacted with the recombinant retroviral vector, either *in vivo* or *ex vivo.* Suitable retroviral vectors include, but are not limited to, those derived from the murine retrovirus M-MuLV, N2 (a retrovirus derived from M-MuLV), or the double copy vectors designated DCT5A, DCT5B and DCT5C (see WO 90/13641).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. In general, conjugation of the ligand binding molecule preferably does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

Antisense or sense RNA or DNA molecules are generally at least about 5 nucleotides in length, alternatively at least about 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides in length, wherein in this context the term "about" means the referenced nucleotide sequence length plus or minus 10% of that referenced length.

Nucleic acid encoding a DSCR1 modulator polypeptide may also be used in gene therapy. In gene therapy applications, genes are introduced into cells in order to achieve *in vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes *in vivo.* It has already been shown that short antisense oligonucleotides can be imported into cells where they act as inhibitors, despite their low intracellular concentrations caused by their restricted uptake by the cell membrane. (Zamecnik et al., Proc. Natl. Acad. Sci. USA 83:4143-4146 [1986]). The oligonucleotides can be modified to enhance their uptake, e.g. by substituting their negatively charged phosphodiester groups by uncharged groups.

There are a variety of techniques available for introducing nucleic acids into viable cells. The techniques vary depending upon whether the nucleic acid is transferred into cultured cells *in vitro*, or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. The currently preferred *in vivo* gene transfer techniques include transfection with viral (typically retroviral) vectors and viral coat protein-liposome mediated transfection (Dzau et al., Trends in Biotechnology 11, 205-210 [1993]). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, proteins that target intracellular localization and enhance intracellular half-life. The technique of receptor-mediated endocytosis is described, for example, by Wu et al., J. Biol. Chem. 262, 4429-4432 (1987); and Wagner et al., Proc. NatI. Acad. Sci. USA 87, 3410-3414 (1990). For review of gene marking and gene therapy protocols see Anderson et al., Science 256, 808-813 (1992).

DSCR1 modulator polypeptides and nucleic acid molecules of the invention may be used diagnostically for tissue typing, wherein DSCR1 polypeptides may be differentially expressed in one tissue as compared to another, preferably in a diseased tissue as compared to a normal tissue of the same tissue type.

This invention encompasses methods of screening compounds to identify those that modulate DSCR1. Screening assays for antagonist drug candidates are designed to identify compounds that bind or complex with the DSCR1 polypeptide, or otherwise interfere with the interaction of the DSCR1 polypeptides with other cellular proteins, including e.g., inhibiting the expression of DSCR1 polypeptide from cells. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with a DSCR1 polypeptide under conditions and for a time sufficient to allow these two components to interact.

In binding assays, the interaction is binding and the complex formed can be isolated or detected in the reaction mixture. In a particular embodiment, the DSCR1 polypeptide or the drug candidate is immobilized on a solid phase, e.g., on a microtiter plate, by covalent or non-covalent attachments. Non-covalent attachment generally is accomplished by coating the solid surface with a solution of the DSCR1 polypeptide and drying. Alternatively, an immobilized antibody, e.g., a monoclonal antibody, specific for the DSCR1 polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a DSCR1 polypeptide, its interaction with DSCR1 can be assayed by methods well known for detecting protein-protein interactions. Such assays include traditional approaches, such as, e.g., cross-linking, co-immunoprecipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. NatI. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER^{™}) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of DSCR1 and other intra- or extracellular components can be tested as follows: usually a reaction mixture is prepared containing DSCR1 and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the DSCR1 polypeptide may be added to a cell along with the compound to be screened for a particular activity and the ability of the compound to inhibit the activity of interest in the presence of the DSCR1 polypeptide indicates that the compound is an antagonist to the DSCR1 polypeptide. The DSCR1 polypeptide can be labeled, such as by radioactivity, such that the number of DSCR1 polypeptide molecules bound to a receptor molecule can be used to determine the effectiveness of the potential antagonist.

A potential DSCR1 antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence which encodes the mature DSCR1 protein can be used to design an antisense RNA oligonucleotide of from about 10 to 40 base pairs in length. A DNA oligonucleotide is designed to be complementary to a region of the gene involved in transcription (triple helix - see Lee et al., Nucl. Acids Res., 6:3073 (1979); Cooney et al., Science, 241: 456 (1988); Dervan et al., Science, 251:1360 (1991)), thereby preventing transcription and the production of the DSCR1 polypeptide. The antisense RNA oligonucleotide hybridizes to the mRNA *in vivo* and blocks translation of the mRNA molecule into the DSCR1 polypeptide (antisense - Okano, Neurochem., 56:560 (1991); Oligodeoxynucleotides as Antisense Inhibitors of Gene Expression (CRC Press: Boca Raton, FL, 1988). The oligonucleotides described above can also be delivered to cells such that the antisense RNA or DNA may be expressed *in vivo* to inhibit production of the DSCR1 polypeptide. When antisense DNA is used, oligodeoxyribonucleotides derived from the translation-initiation site, e.g., between about -10 and +10 positions of the target gene nucleotide sequence, are preferred.

Potential antagonists include small molecules that bind to the active site, the protein interaction site, or other relevant binding site of the DSCR1 polypeptide, thereby blocking the normal biological activity of the DSCR1 polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, e.g., Rossi, Current Biology, 4:469-471 (1994), and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, e.g., PCT publication No. WO 97/33551, *supra*.

These small molecules can be identified by any one or more of the screening assays discussed hereinabove and/or by any other screening techniques well known for those skilled in the art.

In one embodiment, internalizing antibodies are preferred. Antibodies can possess certain characteristics, or modified to possess such characteristics, that enhance delivery of antibodies into cells. Techniques for achieving this are known in the art. In yet another embodiment, an antibody can be expressed in a target cell by introducing a nucleic acid capable of expressing the antibody into a targeted cell. See, e.g., US Pat. Nos. 6,703,019; 6,329,173; and PCT Pub. No. 2003/077945. Lipofections or liposomes can also be used to deliver the antibody into cells. Where antibody fragments are used, the smallest inhibitory fragment that specifically binds to the binding domain of the target protein is generally advantageous. For example, based upon the variable-region sequences of an antibody, peptide molecules can be designed that retain the ability to bind the target protein sequence. Such peptides can be synthesized chemically and/or produced by recombinant DNA technology. See, e.g., Marasco et al., Proc. NatI. Acad. Sci. USA, 90: 7889-7893 (1993).

Entry of modulator polypeptides into target cells can be enhanced by methods known in the art. For example, certain sequences, such as those derived from HIV Tat or the Antennapedia homeodomain protein are able to direct efficient uptake of heterologous proteins across cell membranes. See, e.g., Chen et al., Proc. Natl. Acad. Sci. USA (1999), 96:4325-4329.

The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The following are examples of the methods and compositions of the invention. It is understood that various other embodiments may be practiced, given the general description provided above. The examples are offered for illustrative purposes only, and are not intended to limit the scope of the present invention in any way.

### EXAMPLES

We conducted a genome-wide analysis of genes that are regulated by an angiogenic factor in endothelial cells and identified DSCR1 to be one of the most significantly induced genes. Consistent with an antagonistic function on calcineurin (CnA) signaling, expression of DSCR1 in endothelial cells blocked dephosphorylation, nuclear translocation, and activity of nuclear factor of activated T cell (NFAT), a transcription factor involved in mediating CnA signaling. DSCR1 was not only induced by VEGF, but also by other compounds activating CnA signaling, suggesting a more general role for DSCR1 in activated endothelial cells. As shown herein, transient expression of DSCR1 attenuated a variety of inflammatory marker genes, identifying a previously unknown regulatory role for DSCR1 in activated endothelial cells. Knock-down of endogenous DSCR1 increased NFAT activity and stimulated expression of inflammatory genes on activated endothelial cells. Thus, the negative regulatory feedback loop between DSCR1 and CnA signaling in endothelial cells identified represents a potential molecular mechanism underlying the expression of inflammatory genes following activation of endothelial cells, for example activation by VEGF through VEGFR2.

Vascular endothelial growth factor (VEGF) binds to 2 transmembrane tyrosine kinase receptors, termed VEGFR-1 and -2, which regulate diverse signal transduction pathways including phospholipase C -γ, phosphatidylinositol 3 (PI-3) kinase/AKT, Ras guanosine triphosphatase-activating protein, and the Src family, thereby controlling a variety of vascular functions (for a review, see Ferrara and Gerber¹). VEGF represents the key regulator of physiologic and pathologic angiogenesis associated with a variety of diseases including tumor growth, chronic inflammation, and diabetic retinopathy. In general, interference with VEGF activity in malignancies associated with pathologic angiogenesis frequently slowed down disease progression, was well tolerated, and did not significantly affect healthy, mature vasculature (for a review, see Ferrara et al²). VEGF is also known as VPF- vascular permeability factor - based on its ability to perturb vascular integrity and induce vascular leakage³⁶, ³⁷. VEGF mutants that bind selectively to VEGFR-2 act as endothelial cell mitogens and permeability-enhancing agents whereas VEGFR-1 selective VEGF mutants do not appear to have these activities. VEGF signaling through VEGFR-2 and vascular perturbation appear to be a major pathway for increased vascular leakage thought to be associated with some uses of VEGF in therapeutic angiogenic settings. Identification of novel genes specifically regulated by VEGF in endothelial cells has lead to identification of genes differentially expressed in pathologic versus normal vasculature.

We used a well-established in vitro endothelial cell survival assay, in which addition of VEGF potently suppressed endothelial cell apoptosis induced by serum starvation. This assay has been particularly useful in identifying molecular components of signaling pathways in endothelial cells. For example, this assay system was instrumental in the identification of the crucial role of the PI-3 kinase/Akt signal transduction pathway and the importance of Bcl2 for endothelial cell survival.^{3,4} Similar conditions were selected for the current study because they mimic the remarkable dependency of endothelial cells for VEGF during neoangiogenesis, as observed in preclinical studies in vivo. Using the GeneCalling technology (CuraGen, Branford, CT), we sought to detect VEGF target genes in the entire human endothelial transcriptome.⁵

Here we describe the identification of DSCR1 as a novel VEGF target gene, and the functional characterization of DSCR1 as an anti-inflammatory signaling molecule in endothelial cells. The human gene (DSCR1) is one of 50 to 100 genes that reside within the minimal region on human chromosome 21 (HC21), which, when present in more than 2 copies, causes mental retardation, collectively known as Down syndrome (DS).⁶ DSCR1 belongs to a family of conserved proteins, also termed calcipressins^{38,39,40} or modulatory calcineurin-interacting proteins (MCIPs), comprising 3 members in humans, DSCR1/MCIP1, ZAKI-4/DSCR1L1/MCIP2, and DSCR1L2/MCIP3, that share in common the capacity to bind to calcineurin. DSCR11 functions as a small cytoplasmic signaling molecule regulated by the Ca²⁺/calmodulin-dependent serine/threonine protein phosphatase 2B (PP2B) or calcineurin A (CnA). Consistent with a negative regulatory feedback loop, CnA activity is suppressed by association with DSCR1. DSCR1 is highly expressed in the developing central nervous system and heart, whereas in adults, moderate expression has been detected in numerous tissues and cell types, including striated muscle cells and cardiomyocytes.⁶ Among these, DSCR1 was shown to interfere with CnA signaling in striated muscle cells⁷ and to inhibit cardiac hypertrophy in animal models in vivo.⁸ Minami et al (J. Biol. Chem. (2004), 279(48):50537-50554) has also reported that DSCR1 can be induced by thrombin. Chronic overexpression of DSCR1 has also been associated in disease, such as Alzheimer's disease, and vascular inflammation and vascular perturbation have been implicated in Alzheimer's disease, vascular Dementia, mixed dementia, etc. Ermak et al., J. Biol. Chem. (2001), 276(42):38787-38794,⁴¹,⁴³. Townsend et al.,Ann. N.Y. Acad. Sci. (2002)977:65-76; and Iadecola et al., Stroke, (2003)34;335-337.

CnA plays a crucial role during cellular responses to various extracellular signals and environmental stresses and is important in the regulation of apoptosis, memory processes, and skeletal and cardiac muscle growth and differentiation (for reviews, see Rothermel et a.1⁹ and Crabtree and Olson¹⁰). Dephosphorylation of the nuclear factor of activated T-cell transcription factor (NFATc1) by CnA promotes its translocation to the nucleus, where it acts cooperatively with other transcription factors, including members of the AP1, cMAF, GATA, or MEF2 families.¹¹ The 4 members of this gene family (NFATc1-c4) are expressed in many tissues and display crucial roles during neuronal guidance, skeletal and cardiac muscle hypertrophy, and cardiac valve development (for a review, see Hill-Eubanks et al¹²). In addition to their regulatory roles in inflammatory cells, recent gene ablation experiments in mice suggested independent vascular functions for some members of the NFAT transcription factor family. These findings suggested that NFAT transcription factors may not be required for endothelial cell survival, but play important roles for correct vessel assembly during vessel maturation_{.}¹³⁻¹⁵

Previous studies demonstrated phosphorylation and nuclear translocation of NFAT in endothelial cells exposed to agents stimulating CnA signaling such as phorbol¹²-myristate ¹³-acetate (PMA) and ionomycine (10).^{16,17} Cyclosporin A (CsA) is a potent inhibitor of CnA and based on the inhibitory effects of CsA on gene expression, several potential CnA target genes were described in activated endothelial cells, including granulocyte-macrophage colony-stimulating factor (GM-CSF),¹⁶ Cox-2,¹⁸ interleukin 8 (IL-8),¹⁹ and tissue factor (TF).²⁰ Thus, although several potential CnA target genes were identified in endothelial cells and antagonistic activities of DSCR1 on CnA signaling were described in lymphocytes and cardiomyocytes, the role of DSCR1 in CnA signaling in endothelial cells was not known.

The present study identifies DSCR1 as a novel endogenous inhibitor of CnA in activated endothelial cells and describes the biologic consequences of modulation of DSCR1 levels on inflammatory marker gene expression in endothelial cells. For example, as shown herein, interference with endogenous DSCR1 increased NFAT activity and stimulated expression of several markers of inflammation, demonstrating that modulation of endogenous levels of DSCR1 are sufficient to affect vascular inflammation.

### Materials and methods

### Cloning of human DSCR1

DSCR1 was cloned by polymerase chain reaction (PCR) using a commercially available expression sequence tagged (EST) clone (Incyte, Palo Alto, CA) using primers designed to introduce a *Cla*I site at the 5' end (DSCR1-5'*Cla*I) and an *Eco*R1 site at the 3' terminus (DSCR1-3'*EcoR*1). The PCR fragment was cloned into a cytomegalovirus (CMV)-driven expression vector PRKN (Genentech, South San Francisco, CA) cut with *Cla*I and *Eco*RI and the inserts were verified by sequencing before subjected to further experiments. At the C-terminus, a FLAG epitope was introduced additionally (DSCR1-Flag).

*Reagents*. All primary antibodies, unless indicated otherwise, were obtained from BD Pharmingen (San Diego, CA), recombinant human VEGF (rhVEGF) was obtained from Genentech, and basic fibroblast growth factor (b-FGF) and tumor necrosis factor α (TNF-α) were purchased from R&D Systems (Minneapolis, MN).

*Cells and cell assays*. Primary human endothelial cells were purchased from Clonetics (Santa Rosa, CA) and cultured according to the manufacturer's instructions. For gene calling experiments, human umbilical vein endothelial cells (HUVECs) were purchased from Clonetics (BioWhittaker, Walkersville, MD) and maintained in endothelial growth medium (EBM-2) complemented with 5% fetal calf serum (FCS) following the manufacturer's instructions. Cells (1 x 107) were split at a cell density of 19 000 cell/cm2 and triplicate experiments for each condition were run in parallel. Twenty-four hours after plating the cells, cell were washed 3 times with phosphate-buffered saline (PBS) and media (Clonetics) containing either 0.1% bovine serum albumin (BSA) or 0.1% BSA plus VEGF (30 ng/mL) or 5% FCS. RNA isolation and reverse transcription-PCR (RT-PCR) analysis was performed as described.²¹ For cell culture experiments, the following conditions were used: human (h) VEGF, 30 ng/mL; hTNF-α, 10 ng/mL; PMA, 200 ng/mL (Calbiochem, LaJolla, CA); CsA, 1 µM (Calbiochem); IO, 5 µM (Sigma, St Louis, MO); thapsigargin, 50 nM (Sigma). In one instance inhibiting (si) RNAs were generated by Dharmacon (Lafayette, CO) based on the full-length cDNAs of the respective gene and using the "Smart-pool" service (Dharmacon, Lafayette, CO). For adenoviral transduction survival experiments, human umbilical vein endothelial cells (HUVECs) were maintained in endothelial growth medium (EBM-2) complemented with 5% FCS following the manufacturer's instructions. 3 x 105 cells were split per well of a 6 well plate (Primaria, BD Biosciences, Bedford, MA) and duplicate experiments for each condition were run in parallel. Cells were infected with adenovirus (MOI=100) encoding for a gD peptide (Ad-Control), DSCR1 (Ad-DSCR1) or an antisense construct with the coding sequence inverted (Ad-DSCR1-AS) for 2 h in serum free media containing 10 mM CaCl2 and 10nM MgCl2 on a vertical shaker at room temperature. Thereafter, 5 times the volume of regular growth medium containing 5% FCS was added and cells were kept for 1 day in a CO2 incubator. Thereafter, cells were washed three times with PBS and basal media (Clonetics, Santa Rosa, CA) containing 0.1 % BSA was added and PMA/IO, H2O2, and ZVAD as indicated. 24 hours after serum starvation and dosing, cell were counted on a Coulter brand style cell counter. For cell culture experiments, the following conditions were used: PMA: 200ng/ml (Calbiochem, LaJolla, CA), Ionomycine: 5 µM (Sigma, St. Louis, MO), a 3% H2O2 solution was diluted 1:300 in basal media, 0.1% BSA to result in a 0.01% H2O2 solution, ZVAD 5 µM.

### Generation of adenoviral vectors and transduction of endothelial cells

Ad-DSCR1-Flag and Ad-LacZ were constructed by cloning the *Not*I-*Not*I cDNA insert into the polylinker site of the Ad-easy vector construction kit from Stratagene (La Jolla, CA), essentially as described by the manufacturer.

### Gene Calling

To identify novel genes that are differentially expressed, "open" technologies such as serial analysis of gene expression (SAGE) and GeneCalling were used. GeneCalling has been described elsewhere.^{5,22}

Samples from each treatment group were reverse-transcribed, and subjected to quantitative expression analysis (QEA), .⁵ Analyses focused on identification of genes regulated at least 2-fold. Initial analyses exploring condition-dependent gene expression profiles were carried out by QEA or GeneCalling, as described previously.⁵

### Western blotting and immunoprecipitation

Cells cultured in 10-cm dishes were washed twice in Tris (tris(hydroxymethyl)aminomethane)-buffered saline, and 0.6 mL RIPA buffer (10 mM PBS, 1% Nonidet P-40, 0.5% sodium deoxycholate, 0.1% sodium dodecyl sulfate [SDS], 100 mg/mL phenylmethylsulfonyl fluoride, 30 µg/mL aprotinin, 1 mM sodium orthovanadate), and protease inhibitors (Roche Pharmaceuticals, Gaithersburg, MD) were added. Mouse monoclonal antibody (mAb) directed against human NFATc1, c2, and c3 was purchased from Santa Cruz Biotechnology (Santa Cruz, CA). Affinity-purified goat polyclonal antibody against human E-selectin, vascular cell adhesion molecule-1 (VCAM-1), intercellular adhesion molecule-1 (ICAM-1), and Cox-2 was from BD Pharmingen. Total endothelial cell extract, polyacrylamide gel electrophoresis (PAGE), blotting, and immunodetection were performed according to the Santa Cruz Biotechnology protocol. A total of 60 mg whole-cell extract was loaded on each lane of a 4% to 16% polyacrylamide gel. The enhanced chemiluminescence (ECL) reaction kit was purchased from Amersham Pharmacia Biotech (Piscataway, NJ), and horseradish peroxidase streptavidin from Vector Laboratories (Burlingame, CA) was used. For NFATc1 experiments, equal amounts of cell extract were loaded to visualize NFATc1 (mAb 7A6; 1:500; Santa Cruz Biotechnology) and the loading control α -tubulin (mAb from ICN, Santa Ana, CA; 1:5000). For Cox-2 and TF immunoblot analysis equal amounts of cell lysate were loaded on a 6% to 12% gradient SDS-PAGE. Cox-2 was analyzed with an anti-Cox2 mAb (R.DI, 1:500) and TF with an anti-hTF mAb (clone 7G11) was generated at Genentech, and used at a 1:500 dilution. Before loading the samples on the gel, the protein amounts were quantified and adjusted accordingly. As loading control, an anti-γ-adaptin mAb was used (1:1000) on a 4% to 20% polyacrylamide gel.

### Analysis of endothelial cell apoptosis

For fluorescence-activated cell sorting (FACS) analysis, cells were stained by fluorescein isothiocyanate-conjugated annexin V and by the fluorescent dye propidium iodide (PI) essential as described in Gerber et al. J Biol Chem. 1998;273: 30336-30343.

### Transient transfection of primary human endothelial cells and luciferase assay

For HUVEC and human microvascular endothelial cell (HMVECs) transient transfections, cells (7.5 x 104) were plated per well of a 6-well dish in 3 mL growth medium (Primaria; BD Biosciences, Bedford, MA). The amounts of plasmid DNA used in the lipofection mixture were calculated for transfection of the total of 3 wells. Then, 1.25 µg expression vector, 3.75 µg luciferase reporter, and 2.0 µg SV-Renilla reference reporters were mixed and 14 µL F1 lipofectin reagent (Targeting Systems, Santee, CA) was added. Then, 4.5 mL high-glucose Dulbecco modified Eagle medium (DMEM) was added to the mixture. Transient transfection was conducted as recommended by the manufacturer. For reporter gene expression experiments, cells were lysed by using the Dual Luciferase Reporter Kit (Promega, San Luis Obispo, CA) in 300 µL of 1 x passive lysis buffer following the recommendations of the manufacturer.

For transient transfection experiments including siRNA, HUVECs were plated in 6-well plates as described for transient transfection experiments. For 6 replicas, 600 pmol siRNA (Dharmacon, Lafayette, CO), 7.5 µg luciferase, and 4 µg Renilla reporter construct were mixed in 6 mL high-glucose DMEM. For FACS and immunohistochemical (IHC) experiments, 11.5 µg enhanced green fluorescent protein (EGFP) plasmid was added instead of the luciferase plasmid. Prior to incubation at 37° C for 25 to 30 minutes, 30 µL Targefect solution A and 60 µL Targefect solution B (Targeting Systems) were added to the mixture; 1 mL of this solution was used for lipofection as described. Cells were washed twice with PBS prior to addition of serum-free medium and dosing for the indicated amount of time.

### siRNA Antagonists of DSCRI and NFATC1

| Gene Name Accession Number Based On Sequence of Senses Strand 5'-> 3' | | |
|---|---|---|
| Used as pool in angiogenic factor (e.g., VEGF) activation studies: | | |
| DSCR1 | NM_004414 | GCUCAGACCUACACAUAG |
| DSCR1 | NM_004414 | GGACAUCACCUUUCAGUAU |
| DSCR1 | NM_004414 | GAAAGAAUGAGGAGACCUA |
| DSCR1 | NM_004414 | GACAUCACCUUUCAGUAUU |

| Used in PMA/IO activation studies: | | |
|---|---|---|
| DSCR1 | NM_004414 | AACGUAUGACAAGGACAUCAC |
| NFATC1 | NM_172390, | CGUAUGAGCUUCGGAUUGA |
| NFATC1 | NM_172390, | GCAGAGCACGGACAGCUA |
| NFATC1 | NM_172390, | UCAGAAACUCCGACAUUGA |
| NFATC1 | NM_172390, | GCCGGAAUCCUGAAACUCA |

| | | |
|---|---|---|
| *Note*: *All the siRNAs listed have UU as 3'-overhallgs on each strand and 5'-phosphate on the antisense strand only.* | | |

### Immunofluorescence microscopy

Primary human endothelial cells (7 x 103 cells/cm²) were seeded into each well of an 8-chamber microscope slide (Nalgene Nunc, Naperville, IL) 24 hours prior to staining. Cells were infected with adenovirus (multiplicity of infection [MOI] = 100) encoding for LacZ or DSCR1 for 2 hours in serum-free media containing 10 mM CaCl2 and 10 nM MgCl2 on a vertical shaker at room temperature. Thereafter, 5 times the volume of regular growth medium was added and cells were kept for 2 days in a CO2 incubator. After replacing the media, cells were treated with hVEGF at a concentration of 30 ng/mL for 20 minutes. Prior to incubation with various antibodies, cells were fixed with methanol for 4 minutes at -20° C and washed with PBS at room temperature. Cells were incubated in PBS containing the primary mouse antihuman NFATc1 antibody (sc7294, Santa Cruz Biotechnology) for 1 hour at room temperature. Cells were then washed 3 times with PBS and incubated in PBS containing Alexa Fluor 488 antimouse IgG (Molecular Probes, Eugene, OR). Cells were finally washed 3 times with PBS and mounted beneath coverslips in Vectashield medium (Vector Laboratories). Microscopy and photography were conducted using a Zeiss Axiovert 200 fluorescence microscope fitted with an AxioCam MR monochrome camera (Zeiss, Thornwood, NY) using a x 63 oil objective.

### Generation of polyclonal antibodies for human DSCR1 in rabbits and Western blot analysis

DSCR1 was PCR amplified with primers DSCR1-EcoR1-Pro 5', adding an extra proline and DSCR1-Stop-XhoI 3', by using DSCR1 cDNA as a template and cloned into pGEX-KG. GST-DSCR1 was expressed and purified essentially as described previously.²³ Polyclonal rabbit anti-GST-DSCR1 was prepared by immunization with recombinant GST-DSCR1. Filtered crude serum was used for Western blots (1:400). Oligo and *Taq*Man probe primer sets are shown in Table 1.

**Table 1. Oligo and TaqMan probe primer sets**

| | Primer set |
|---|---|
| HSDSCR1.1113FP | 5' (FAM) -AGG TTG TGA AAA CAG CAG CAA TGC AAT GT- (TAMRA) P3' |
| HSDSCR1.1088.F | CCA CAG GAA GCC GCC TAG T |
| HSDSCR1.1171.R | TGA GGG AAG AAA GGA AAC GCT |
| HSGMCSF-571T | 5'(FAM) -AGG TCC AGG CCA CTC CCA CC GT- (TAMRA) P3' |
| HSGMCSF-515F | GTC ATC TTG GAG GGA CCA A |
| HSGMCSP-610R | TTC TGC CAT GCC TGT ATC A |
| HSTF-1911T | 5' (FAM) -TTA ACT GAC TTA AGT GGC ATT AAA CAT TTG AGA GCT AA GT- (TAMRA) P3' |
| HSTF-1880F | CAG CTT CCT AAT ATG CTT TAC AAT CT |
| HSTF-2025R | CAA TGT CAA CCA TAG AAG CTT TAA GTA |
| HSCOX-2-3152T | 5'(FAM) -CCT GGC TAC CTG CAT GCT GTT CC GT- (TAMRA) P3' |
| HSCOX-2-3130F | CAG TAG GTG CAT TGG AAT CAA |
| HSCOX-2-3207R | TCT TAG CAA AAT GGC TAA AAG AAG |
| HSCD34-2497T | 5'(FAM) -ATC CCT GCT CAA CCC CTC TGG A GT- (TAMRA) P3' |
| HSCD34-2447P | CCC AGG TCC TTG TTT GCT |
| HSCD34-2519R | TCC AAC CGT CAT TGA AAC C |
| HSVCAM-1-2686T | 5' (FAM) -CGA AGT TTG TTC ATC AGA CTC CTG TGC GT- (TAMRA) P3' |
| HSVCAM-1-2665F | GCA TGG TAC GGA GAT GTT TC |
| HSVCAM-1-2730R | GGC CAC ATT GGG AAA GTT |
| DSCR1-EcoRI-Pro 5' | CCGAATTCCCATGCATTTTAGAAACTTTAACTACAG |
| DSCR1-Stop-XhoI 3' | GGGCTCGAGCTAGCTGAGGTGGATCGGCGTGTACTCC |

### Results

### Identification of novel VEGF target genes

Genes specifically regulated by VEGF in the absence of serum were identified by differential electronic subtraction of gene expression profiles derived from HUVECs stimulated with VEGF (30 ng/mL) or 5% FCS relative to basal conditions (no addition [NA]), respectively. A visual display of such analysis is shown in Figure 1A, demonstrating that VEGF induced DSCR1 mRNA expression in HUVECs 4.8-fold after 6 hours and 2.9-fold after 24 hours of stimulation, respectively. The presence of 5% FCS did not significantly alter expression levels of DSCR1. Overall, we detected changes in 1.8% and 4.4% of traces representing alterations in expression of genes in FCS-stimulated cells after 6 and 24 hours, respectively. For VEGF-stimulated cells, we observed changes in 1.7% and 3.4% of genes after 6 and 24 hours, respectively. The identities of 46 cDNA fragments specifically induced by VEGF but not FCS were determined as described previously Twenty-eight cDNAs corresponded to known genes or ESTs and among these, DSCR1 was most strongly induced. The remaining 18 cDNA fragments did not match any known sequences within the public database. Thirteen (46%) of the 28 known genes belong to the class of secreted factors, which further emphasizes the crucial role of the endothelium as secretory tissue.²⁴ A total of 6 genes (21%) belong to the class of cytoplasmic proteins, potentially involved in signal transduction, including DSCR1. The remaining 9 genes (37%), comprising 3 ribosomal, 2 mitochondrial, 2 transcription factors, 1 cell cycle regulatory gene, and one gene involved in regulation of translation, represent the class of genes involved in metabolic and cell cycle control.

### Conditions leading to activated endothelial cells induce DSCR1 expression

To verify the results obtained from GeneCalling technology, we analyzed RNA levels in various human endothelial cells stimulated with VEGF or other endothelial cell mitogens, by real-time RT-PCR analysis (*Taq*Man). Confirming our previous results (Figure 1A), addition of FCS or b-FGF to serum-starved HUVECs did not alter DSCR1 levels significantly, whereas addition of VEGF induced prolonged expression of DSCR1 in HUVECs over 36 hours (Figure 1B). These findings confirmed the data from GeneCalling experiments and suggested that DSCR1 is not a general marker expressed in mitotic endothelial cells.

To evaluate the extent to which each of the 2 VEGFRs is mediating the induction of DSCR1, we tested mutant VEGF forms, which activate each VEGFR in an exclusive manner, as described previously.^{25,26} The VEGFR-2 selective mutant form (VEGFR2-sel) was equally potent to VEGF in inducing DSCR1 expression. In contrast, VEGFR-1 selective ligands (VEGFR1-sel and PIGF) failed to induce alterations in DSCR1 expression, indicating VEGFR-2 to be sufficient to induce DSCR1 expression (Figure 1B-C).

Endothelial cells isolated from different tissues may vary in their levels of VEGFR expression or in their signal transduction pathways, which may affect their VEGF target gene expression profiles. To test for such potential cell type-specific differences, we stimulated human pulmonary aortic endothelial cells (HPAECs), human dermal microvascular endothelial cells (HMVECs), and human aortic endothelial cells (HUAECs) with VEGF or receptor selective mutant forms and analyzed DSCR1 expression by realtime RT-PCR. As a negative control, we tested human pulmonary aortic smooth muscle cells (HPASMCs), which do not express significant levels of VEGF receptors (data not shown). All endothelial cells tested displayed increased levels of DSCR1 expression in response to stimulation by VEGF and VEGFR2-sel but not VEGFR1-sel, indicating the VEGFR-2 was necessary and sufficient for induction of DSCR1 in a variety of endothelial cells (Figure 1C and data not shown). In general, most cell types displayed a less prominent increase in DSCR1 expression after prolonged stimulation by VEGF when compared to HUVECs (Figure 1B).

To assess whether conditions generally leading to activated endothelial cells induce DSCR1 expression and whether CnA is involved in the regulation of DSCR1, we stimulated HUVECs with TNF-α or compounds activating CnA signaling such as the calcium ionophore A23187 [GenBank] (IO), alone or in combination with PMA and thapsigargin, another activator of CnA signaling. All compounds significantly increased DSCR1 mRNA levels after 5.5 hours (Figure 1D) and 18 hours (data not shown) and such induction was potently repressed in presence of CsA, an inhibitor of CnA signaling. Interestingly, TNF-α induced DSCR1 to similar levels as observed for VEGF. Western blot analysis of whole cell extract prepared from HUVECs confirmed induction of DSCR1 following 6 hours of stimulation by VEGF, PMA/IO, and thapsigargin (Figure 1E). Similar to the findings for mRNA levels, no significant increase in DSCR1 protein levels were found following stimulation with b-FGF or 5% FCS. In contrast to the findings for mRNA levels, we were unable to detect increased DSCR1 protein levels in cells stimulated with TNF-α, suggesting a different mechanism of regulation of DSCR1 expression by VEGF and TNF-α. Based on the inhibitory effects of CsA on DSCR1 levels on activated endothelial cells, we conclude that this induction was mostly mediated by CnA. However, the incomplete repression of mRNA expression by CsA following stimulation with TNF-α suggests the presence of additional, unknown regulatory mechanism in endothelial cells.

### DSCR1 prevents nuclear translocation of NFAT

VEGF, when added to human pulmonary vein endothelial cells (HPVECs), induced nuclear translocation of NFATc1 as early as 20 minutes after stimulation.²⁷ Thus, we wanted to analyze the kinetics of the nuclear translocation of NFATc1 in HUVECs and to what extent DSCR1 may interfere with this process. IHC analysis of endothelial cells revealed prominent nuclear localization of NFATc1 in cells stimulated by VEGF, which was absent in cells expressing DSCR1 20 minutes after stimulation (Figure 2A) and also after prolonged incubation for 60 and 120 minutes (data not shown). This, to our knowledge, is the first demonstration of a functional interference between DSCR1 and NFAT in activated primary human endothelial cells, preventing nuclear translocation of NFATc1. Additionally, we studied subcellular localization of DSCR1 in endothelial cells after adenoviral transduction with vectors encoding epitope-tagged DSCR1 (Ad-DSCR1-FLAG) or control vector (Ad-LacZ) by IHC methods. In agreement with previous reports,^{7,28,41} we detected cytoplasmic and nuclear localization of DSCR1 after transduction with Ad-DSCR1-FLAG, which was unaltered in the presence of VEGF or IO/PMA (data not shown).

Dephosphorylation of NFATc1 by the serine threonine phosphatase CnA generates the activated form of this transcription factor, capable of entering the nucleus and stimulating transcription.²⁸ Thus, we wanted to compare the effects of DSCR1 on the phosphorylation status of NFATc1 in endothelial cells with the effects induced by CsA. Similar to the findings by Johnson et al,²⁷ analysis of cell extracts of activated endothelial cells demonstrated a shift from the dephosphorylated form of NFATc1 to the phosphorylated form in response to DSCR1 (labeled with "P" in Figure 2B). These changes were comparable to the effects induced by CsA and suggest blocking of the phosphatase activity of CnA. In addition, overall NFATc1 protein levels were increased by DSCR1, indicating that DSCR1 expression may affect NFATc1 protein stability. In contrast, CsA did not significantly alter NFATc1 protein levels, suggesting potentially different mechanisms of action between CsA and DSCR1 regulating CnA activity and NFATc1 phosphorylation.

### DSCR1 attenuates transcriptional activity of NFAT

Experiments with transformed tumor cells demonstrated functional interference between DSCR1 and NFAT transcriptional activity in cells exposed to compounds stimulating CnA such as IO and PMA.²⁸ To test whether such a mechanism was present in endothelial cells, we transiently cotransfected expression vectors for DSCR1 and quantified the levels of NFAT activity by analysis of luciferase reporter gene expression in endothelial cells. Similar to the effects induced by CsA, expression of DSCR1 strongly repressed NFAT-driven transcription in endothelial cells stimulated with PMA, thapsigargin, TNF-α , or the calcium ionophore A23187 [GenBank] (Figure 3A). DSCR1 did not interfere significantly with AP1 activity (Figure 3B), demonstrating that the effects were specific for NFAT.

### DSCR1 suppresses expression of inflammatory maker genes on activated endothelial cells

To study the effects of increased expression of DSCR1 on CnA target gene expression, we transduced endothelial cells with adenoviral vectors encoding for DSCR1 and analyzed CnA target gene expression levels following activation. The conditions tested included 4 hours of incubation with VEGF, PMA/IO or thapsigargin and combinations thereof. Analysis of mRNA levels by real-time RT-PCR revealed significant induction of COX2, E-selectin, and TF in activated endothelial cells (Figure 4A). In agreement with previous reports, the 2- to 3-fold increase in response to stimulation by VEGF was markedly below the 10- to 20-fold increase in gene expression induced by IO/PMA.²⁰ These differences probably reflect that PMA, acting intracellularly, is a more potent inducer of CnA signaling relative to VEGF, which binds to VEGFR2 expressed extracellularly. As demonstrated here for the first time, expression of DSCR1 repressed inflammatory marker gene expression in endothelial cells stimulated by VEGF or IO/PMA.

To verify whether these changes in RNA levels correlate with alterations in protein levels, we conducted a series of FACS and Western blotting experiments. The data shown in Figure 4B-C demonstrate down-regulation of E-selectin, VCAM-1, TF, and Cox-2 by DSCR1. The reductions in TF and Cox-2 protein levels were more pronounced in response to DSCR1 expression when compared to the effects induced by CsA alone (Figure 4C, compare lanes 4 and 7 with 4 and 5, and 6 and 9 with 6 and 5, respectively). These findings demonstrate that DSCR1 reduces expression of several inflammatory maker genes more potently than CsA in activated human endothelial cells.

Next, we monitored the expression kinetics of VEGF target genes, including *DSCR1*, in response to VEGF stimulation. As expected, GM-CSF, TF, COX-2, VCAM-1, and E-selectin RNA levels were increased between 0 and 2 hours after VEGF administration (Figure 5A). However, such induction was transient and reversed into repression between 6 and 36 hours after stimulation, when compared to control, untreated cells. As a control, we analyzed expression of Bcl-2 in response to VEGF stimulation, which was consistently expressed over the entire experiment (Figure 5A and Gerber et al³). In conclusion, decreased expression of VEGF target genes coincided with increased DSCR1 expression levels in response to VEGF stimulation. The combined data support our model, wherein DSCR1 may act in a negative feedback regulatory loop interfering with CnA signaling in activated endothelial cells (Figure 5B).

### Perturbation using adenovirus DSCR1 modulates survival of endothelial cells

Overexpression of DSCR1 by Ad-DSCR1 in endothelial cells exposed to stress conditions, induced endothelial cell death as did reduction of endogenous DSCR1 by expression of the DSCR1 anti-sense construct. This was seen under serum starvation, H₂O₂ exposure and PMA/IO treatment and in the presence of Caspase inhibitors such as ZVAD specifically.

### Knock down of DSCR1 induced NFAT activity and expression of inflammatory marker genes on activated endothelial cells

To test the effects of transfection of endothelial cells with siRNA, we conducted Western blot analysis of cells cotransfected with an expression vector encoding for an epitope-tagged form of DSCR1 (DSCR1-FLAG) using an anti-Flag antibody. Alternatively, we assessed the endogenous levels of DSCR1 by using rabbit polyclonal antiserum. We found a complete absence of DSCR1 protein expression when an expression vector encoding for a FLAG-tagged version (PRKN-DSCR1-FLAG) was cotransfected (Figure 6A), indicating that siRNA targeting DSCR1 potently reduced DSCR1 expression. As expected, we found a 50% to 70% reduction in endogenous DSCR1 levels, which is consistent with a similar transfection efficiency as assessed by fluorescence microscopy of cells cotransfected a GFP expression vector (data not shown). These data suggested the transient transfection of endothelial cells with siDSCR1 efficiently reduced protein levels in endothelial cells.

Next we applied these conditions to investigate the effects of decreased endogenous DSCR1 levels on CnA downstream signaling events. We transiently cotransfected siRNA with luciferase reporter constructs containing NFAT-binding sites and activated transfected endothelial cells with VEGF or PMA/IO. There was a 2- and 3-fold increase in NFAT activity when cells were transfected with siDSCR1 relative to control transfected cells (siControl) in presence of both, IO/PMA (Figure 6C) or VEGF (Figure 6D). In contrast to siDSCR1-transfected cells, there was a decrease in NFAT activity in siNFATc1-transfected cells, confirming the validity the siRNA cotransfection method for primary human endothelial cells (Figure 6C-D).

Finally, we wished to analyze the effects of knocking down endogenous DSCR1 expression on CnA target genes expression in activated endothelial cells. To select for cells transfected with siRNA, we added a GFP expression vector to the transfection mixture. FACS analysis of GFP+ cells revealed an increase in the expression levels of several inflammatory marker genes, including TF, E-selectin, and VCAM-1 in presence of siDSCR1 (Figure 7A), whereas ICAM-1 expression was not significantly affected (data not shown). In contrast, cotransfection of cells with siNFATc1 blocked gene expression, suggesting a key role of this transcription factor in E-selectin, VCAM-1, and TF expression in activated endothelial cells. Interestingly, basal and activated levels of VCAM-1 were both repressed by siNFATc1, demonstrating a role for NFATc1 in basal expression of VCAM-1. The magnitude of the induction of inflammatory gene expression by siDSCR1 in cells stimulated by VEGF was clearly below the levels observed for cells stimulated with PMA/IO (Figure 7A-B). Similar to other reports,²⁰ these findings further demonstrate a reduced potential of VEGF to induce calcineurin signaling relative to the pharmacologic compounds such as PMA/IO. In summary, our findings demonstrate that endogenous levels of DSCR1 are sufficient to repress expression of inflammatory genes on endothelial cells stimulated by VEGF or other activators of CnA signaling.

### Discussion

### Differences in the mechanism of action between CsA and DSCR1

Although both DSCR1 and CsA interfere with CnA activity, they differ in their molecular targets and their mechanism of interference. CsA and other immunosuppressive drugs like FK506 inhibit calcineurin by forming complexes with specific cellular immunophilins (FKB12 and cyclophilin A, respectively) and this complex binds to multiple sites on calcineurin. In contrast, DSCR1 binds CnA directly via structural motifs that resemble calcineurin interacting domains first identified in NFAT proteins. These conserved motifs within DSCR1 appear to have functional importance because truncated forms of DSCR1, lacking these regions, are defective for binding to CnA⁷ (for a review, see Rothermel et al⁹). Therefore, DSCR1 is believed to directly contact the active site of calcineurin, perhaps by inhibiting access of NFAT and other phosphoprotein substrates.

We analyzed whether the differences in their mechanism of action would translate into different pharmacologic effects. As shown in Figure 4, DSCR1 and PMA/IO significantly reduced expression of several NFAT target genes, including E-selectin, TF, and Cox-2 in endothelial cells stimulated with VEGF, thapsigargin, or PMA/IO. However, the reduction in gene expression and the hyperphosphorylation of NFATc1 induced by DSCR1 was generally more pronounced when compared to the effects induced by CsA (Figure 4C). In conclusion, the increased inhibitory effects of DSCR1 on expression of inflammatory genes relative to CsA appear to be caused by interference of DSCR1 with other signal transduction pathways regulating inflammatory events in endothelial cells. Alternatively, the differential response may be caused by the differences in the pharmacologic properties of CsA, which is added to the culture media as opposed to adenoviral vectors expressing DSCR1 in the cells.

### DSCR1 and the regulation of inflammatory genes on activated endothelial cells

CnA and the downstream transcription factor NFAT likely play a role during developmental angiogenesis. Mice defective in both NFATc1 alleles display defective aortic and pulmonary valve development with subsequent death around embryonic day 14.5, as a consequence of congestive heart failure.^{13,15} Double knock-out experiments in mice lacking NFATc3 and NFATc4 did not impair valve development, but resulted in embryonic lethality around embryonic day 11 due to generalized defects in blood vessel assembly.¹⁴ A similar vascular phenotype was observed in embryos deficient in both CnA alleles. These findings suggested that CnA signal transduction pathways do not directly control endothelial cell survival but are important for vessel maturation.²⁹ In support of this notion, we have not detected any significant changes in endothelial cell survival in response to alterations in DSCR1 levels (data not shown).

### A negative feedback loop by DSCR1 in endothelial cells

In endothelial cells, many of the genes repressed by CsA^{16,18-20} were independently described as being regulated by VEGF.^{16,18-20,30-33} These observations implied that VEGF may regulate this class of genes via CnA. In support of this theory, addition of CsA to endothelial cells stimulated with VEGF attenuated expression levels of IL-8, GM-CSF, and Cox-2.^{18,32-34} All of these genes are up-regulated by VEGF in a transient manner and prolonged incubation with VEGF results in their down-regulation. Our work in endothelial cells points to the concomitant up-regulation of NFATc1, a transcription factor mediating CnA signaling, and the inhibitory protein DSCR1, where DSCR1 generates a negative feedback loop (Figure 5B) in endothelial cells. In support of this signaling pathway, we found highest levels of E-selectin, TF, COX-2, and VCAM-1 expression 2 hours after VEGF stimulation. In contrast, 6 hours after treatment, expression levels of these genes were equal to or below the levels of untreated control cells. During the time course of the experiment, DSCR1 levels increased for the first 6 hours after stimulation and reached a plateau afterward (Figure 5A). Consistent with negative feedback, expression of VEGF target genes inversely correlated with DSCR1 expression.

The model of a negative feedback loop further predicts that knock down of the inhibitory component would result in increased levels of inflammatory marker genes on endothelial cells (Figure 5B). Consistent with this expectation, siRNA experiments targeting endogenous DSCR1 revealed increased NFAT transcriptional activity (Figure 6C) and expression of CnA target genes, including TF, VCAM-1, and E-selectin on activated endothelial cells (Figure 7A). When analyzing endothelial cells treated with VEGF, knock down of endogenous DSCR1 by siRNA resulted in increased activity of NFAT (Figure 6D) and expression levels of TF, E-selectin, and VE-cadherin (Figure 7B). Overall, the magnitude of induction in VEGF-stimulated cells was less pronounced when compared to PMA/IO treatment, which may be explained by the superior potency of the later compounds to stimulate CnA activity.

Concomitant induction of stimulatory and inhibitory signaling events in response to cytokine stimulation has been described for other biologic systems. A negative feedback loop was described in lymphocytes to limit the effects of prolonged stimulation by TNF-α or IL-1. Both cytokines induce A20, an intracellular adaptor molecule that interferes negatively with TNF-α signaling in various cell types.³⁵ 35 Thus, similar to the protective role of A20 in TNF-α signaling, we believe DSCR1 could prevent vascular damage during periods of prolonged endothelial cell activation by angiogenic factors (e.g., VEGF) or other stimuli.

In conclusion, we provide herein the first direct evidence for a role of DSCR1 in the repression of inflammatory genes on endothelial cells stimulated with proinflammatory compounds. Given the prominent role of some of these genes as therapeutic targets, including Cox-2 and TF, our findings point to DSCR1 as a novel therapeutic target to control expression of inflammatory events on the vasculature, and affect the perturbation of vascular integrity

### PARTIAL LIST OF REFERENCES

1. Ferrara N, Gerber HP. The role of vascular endothelial growth factor in angiogenesis. Acta Haematol. 2001;106: 148-156.
2. Ferrara N, Gerber H, LeCouter J. The biology of VEGF and its receptors. Nat Med. 2003;9: 669-676.
3. Gerber HP, Dixit V, Ferrara N. Vascular endothelial growth factor induces expression of the antiapoptotic proteins Bcl-2 and A1 in vascular endothelial cells. J Biol Chem. 1998;273: 13313-13316.
4. Gerber HP, McMurtrey A, Kowalski J, et al. Vascular endothelial growth factor regulates endothelial cell survival through the phosphatidylinositol 3'-kinase/Akt signal transduction pathway. Requirement for Flk-1/KDR activation. J Biol Chem. 1998;273: 30336-30343.
5. Shimkets RA, Lowe DG, Tai JT, et al. Gene expression analysis by transcript profiling coupled to a gene database query. Nat Biotechnol. 1999; 17: 798-803.
6. Fuentes JJ, Pritchard MA, Planas AM, Bosch A, Ferrer I, Estivill X. A new human gene from the Down syndrome critical region encodes a prolinerich protein highly expressed in fetal brain and heart. Hum Mol Genet. 1995;4: 1935-1944.
7. Rothermel B, Vega RB, Yang J, Wu H, Bassel-Duby R, Williams RS. A protein encoded within the Down syndrome critical region is enriched in striated muscles and inhibits calcineurin signaling. J Biol Chem. 2000;275: 8719-8725.
8. Rothermel BA, McKinsey TA, Vega RB, et al. Myocyte-enriched calcineurin-interacting protein, MCIP1, inhibits cardiac hypertrophy in vivo. Proc Natl Acad Sci U S A. 2001;98: 3328-3333.
9. Rothermel BA, Vega RB, Williams RS. The role of modulatory calcineurin-interacting proteins in calcineurin signaling. Trends Cardiovasc Med. 2003; 13: 15-21.
10. Crabtree GR, Olson EN. NFAT signaling: choreographing the social lives of cells. Cell. 2002; 109(Suppl): S67-79.
11. Rao A, Luo C, Hogan PG. Transcription factors of the NFAT family: regulation and function. Annu Rev Immunol. 1997;15: 707-747.
12. Hill-Eubanks DC, Gomez MF, Stevenson AS, Nelson MT. NFAT regulation in smooth muscle. Trends Cardiovasc Med. 2003;13: 56-62.
13. de la Pompa JL, Timmerman LA, Takimoto H, et al. Role of the NF-ATc transcription factor in morphogenesis of cardiac valves and septum. Nature. 1998;392:182-186.
14. Graef IA, Gastier JM, Francke U, Crabtree GR. Evolutionary relationships among Rel domains indicate functional diversification by recombination. Proc Natl Acad Sci U S A. 2001;98: 5740-5745.
15. Ranger AM, Grusby MJ, Hodge MR, et al.. The transcription factor NF-ATc is essential for cardiac valve formation. Nature. 1998;392: 186-190.
16. Cockerill GW, Bert AG, Ryan GR, Gamble JR, Vadas MA, Cockerill PN. Regulation of granulocyte-macrophage colony-stimulating factor and E-selectin expression in endothelial cells by cyclosporin A and the T-cell transcription factor NFAT. Blood. 1995;86: 2689-2698.
17. Alvarez-Arroyo MV, Yague S, Wenger RM, et al. Cyclophilin-mediated pathways in the effect of cyclosporin A on endothelial cells: role of vascular endothelial growth factor. Circ Res. 2002;91: 202-209.
18. Hernandez GL, Volpert OV, Iniguez MA, et al.. Selective inhibition of vascular endothelial growth factor-mediated angiogenesis by cyclosporin A: roles of the nuclear factor of activated T cells and cyclooxygenase 2. J Exp Med. 2001;193: 607-620.
19. Boss V, Wang X, Koppelman LF, Xu K, Murphy TJ. Histamine induces nuclear factor of activated T cell-mediated transcription and cyclosporin A-sensitive interleukin-8 mRNA expression in human umbilical vein endothelial cells. Mol Pharmacol. 1998;54: 264-272.
20. Armesilla AL, Lorenzo E, Gomez del Arco P, Martinez-Martinez S, Alfranca A, Redondo JM. Vascular endothelial growth factor activates nuclear factor of activated T cells in human endothelial cells: a role for tissue factor gene expression. Mol Cell Biol. 1999;19: 2032-2043.
21. Gerber HP, Kowalski J, Sherman D, Eberhard DA, Ferrara N. Complete inhibition of rhabdomyosarcoma xenograft growth and neovascularization requires blockade of both tumor and host vascular endothelial growth factor. Cancer Res. 2000;60: 6253-6258.
22. Quinn-Senger KE, Ramachandran R, Rininger JA, Kelly KM, Lewin DA. Staking out novelty on the genomic frontier. Curr Opin Chem Biol. 2002; 6: 418-426.
23. Smith DB, Johnson KS. Single-step purification of polypeptides expressed in Escherichia coli as fusions with glutathione S-transferase. Gene. 1988;67: 31-40.
24. LeCouter J, Moritz DR, Li B, et al. Angiogenesis-independent endothelial protection of liver: role of VEGFR-1. Science. 2003;299: 890-893.
25. Li B, Fuh G, Meng G, et al. Receptor-selective variants of human vascular endothelial growth factor. Generation and characterization. J Biol Chem. 2000;275: 29823-29828.
26. Gille H, Kowalski J, Li B, et al. Analysis of biological effects and signaling properties of Flt-1 (VEGFR-1) and KDR (VEGFR-2). A reassessment using novel receptor-specific vascular endothelial growth factor mutants. J Biol Chem. 2001;276: 3222-3230.
27. Johnson EN, Lee YM, Sander TL, et al. NFATc1 mediates vascular endothelial growth factor-induced proliferation of human pulmonary valve endothelial cells. J Biol Chem. 2003;278: 1686-1692.
28. Fuentes JJ, Genesca L, Kingsbury TJ, et al. DSCP1, overexpressed in Down syndrome, is an inhibitor of calcineurin-mediated signaling pathways. Hum Mol Genet. 2000;9: 1681-1690.
29. Graef IA, Chen F, Crabtree GR. NFAT signaling in vertebrate development. Curr Opin Genet Dev. 2001;11: 505-512.
30. Clauss M, Gerlach M, Gerlach H, et al. Vascular permeability factor: a tumor-derived polypeptide that induces endothelial cell and monocyte procoagulant activity, and promotes monocyte migration. J Exp Med. 1990;172: 1535-1545.
31. Clauss M, Weich H, Breier G, et al. The vascular endothelial growth factor receptor Flt-1 mediates biological activities. Implications for a functional role of placenta growth factor in monocyte activation and chemotaxis. J Biol Chem. 1996;271: 17629-17634.
32. Kim I, Moon SO, Kim SH, Kim HJ, Koh YS, Koh GY. Vascular endothelial growth factor expression of intercellular adhesion molecule 1 (ICAM-1), vascular cell adhesion molecule 1 (VCAM-1), and E-selectin through nuclear factor-kappa B activation in endothelial cells. J Biol Chem. 2001;276: 7614-7620.
33. Kim I, Moon SO, Park SK, Chae SW, Koh GY. Angiopoietin-1 reduces VEGF-stimulated leukocyte adhesion to endothelial cells by reducing ICAM-1, VCAM-1 and E-selectin expression. Circ Res. 2001;89: 477-479.
34. Kluger MS, Johnson DR, Pober JS. Mechanism of sustained E-selectin expression in cultured human dermal microvascular endothelial cells. J Immunol. 1997;158: 887-896.
35. Jaattela M, Mouritzen H, Elling F, Bastholm L. A20 zinc finger protein inhibits TNF and IL-1 signaling. J Immunol. 1996;156: 1166-1173.
36. Sanger DR, et al. Tumor cells secrete a vascular permeability factor that promotes accumulation of the ascites fluid. Science 1983;219: 983-985.
37. Dvorak, HF, et al., Vascular permeability factor/ vascular endothelial growth factor, microvascular hyperpermeability, and angiogenesis. Am. J. Pathol. 1995;146: 1026-1039.
38. Ermak G. Harris CD. Davies KJ. The DSCR1 (Adapt78) isoform 1 protein calcipressin 1 inhibits calcineurin and protects against acute calcium-mediated stress damage, including transient oxidative stress. FASEB Journal. 16(8):814-24, 2002 Jun.
39. Parry RV. June CH. Calcium-independent calcineurin regulation. Nature Immunology. 4(9):821-3, 2003 Sep.
40. Ryeom S. Greenwald RJ. Sharpe AH. McKeon F. The threshold pattern of calcineurin-dependent gene expression is altered by loss of the endogenous inhibitor calcipressin. Nature Immunology. 4(9):874-81, 2003 Sep.
41. Pfister SC. Machado-Santelli GM. Han SW. Henrique-Silva F. Mutational analyses of the signals involved in the subcellular location of DSCR1. BMC Cell Biology. 3(1):24, 2002 Sep 11.
42. Leahy KP. Crawford DR. adapt78 protects cells against stress damage and suppresses cell growth. Archives of Biochemistry & Biophysics. 379(2):221-8, 2000 Jul 15.
43. Ermak G. Davies KJ. DSCR1(Adapt78)--a Janus gene providing stress protection but causing Alzheimer's disease?. IUBMB Life. 55(1):29-31, 2003 Jan.
44. Vega RB. Yang J. Rothermel BA. Bassel-Duby R. Williams RS. Multiple domains of MCIP1 contribute to inhibition of calcineurin activity. Journal of Biological Chemistry. 277(33):30401-7, 2002 Aug 16.
45. Detmar, VEGF up-regulates DSCR1: more surprises. Blood (2004), 104: 6-7.

## Claims

1. A DSCR1 modulator that inhibits DSCR1 activity in endothelial cells, wherein the DSCR1 modulator is a small inhibitory/interfering RNA (siRNA) comprising one or more sequences selected from the group consisting of
GCUCAGACCUUACACAUAG (SEQ ID NO:2), GGACAUCACCUUUCAGUAU
(SEQ ID NO:3), GAAAGAAUGAGGAGACCUA (SEQ ID NO:4),
GACAUCACCUUUCAGUAUU (SEQ ID NO:5),
AACGUAUGACAAGGACAUCAC (SEQ ID NO:6)
AAGGACAUCACCUUUCAGUAU (SEQ ID NO: 7)
AAGUUAUAUUUUGCUCAGACC (SEQ ID NO:8), and
AAGAUGCGACCCCAGUCAUAA (SEQ ID NO:9),
wherein the modulator induces an increase in a cellular event associated with NFAT activity.

2. The DSCR1 modulator of claim 1, wherein the cellular event is NFAT phosphorylation, NFAT nuclear translation or NFAT transcriptional activity.

3. The DSCR1 modulator of claim 1, wherein the modulator is capable of antagonising DSCR1 function.

4. The DSCR1 modulator of claim 1, wherein the modulator increases the expression of an inflammatory gene.

5. The DSCR1 modulator of claim 4, wherein the inflammatory gene is selected from the group consisting of: TF, VCAM-1 and E-selectin.

6. An in vitro method for inhibiting DSCR1 activity in endothelial cells, the method comprising contacting the cells with a DSCR1 modulator according to any of claims I to 5.
